(19) **Europäisches Patentamt**
**European Patent Office**
**Office européen des brevets**

(11) **EP 3 177 665 B1**

(12) **FASCICULE DE BREVET EUROPEEN**

(45) Date de publication et mention
de la délivrance du brevet:
**05.10.2022   Bulletin 2022/40**

(21) Numéro de dépôt: **15763046.8**

(22) Date de dépôt: **03.08.2015**

(51) Classification Internationale des Brevets (IPC):
**C08G 63/664** *(2006.01)*   **A61L 31/06** *(2006.01)*
**C08L 67/02** *(2006.01)*   **C08L 67/04** *(2006.01)*
**A61K 31/77** *(2006.01)*   **A61P 15/00** *(2006.01)*

(52) Classification Coopérative des Brevets (CPC):
(C-Sets disponibles)
**A61K 31/77; A61L 31/06; A61P 15/00;
C08G 63/664; C08L 67/025; C08L 67/04**   (Cont.)

(86) Numéro de dépôt international:
**PCT/FR2015/052139**

(87) Numéro de publication internationale:
**WO 2016/020613 (11.02.2016 Gazette 2016/06)**

(54) **COMPOSITION DE COPOLYMÈRES DIBLOCS ET TRIBLOCS ET SON UTILISATION DANS LA PRÉVENTION DES ADHÉRENCES TISSULAIRES**

ZUSAMMENSETZUNG VON DIBLOCK- UND TRIBLOCKCOPOLYMEREN UND VERWENDUNG DAVON BEI DER VORBEUGUNG VON GEWEBEADHÄSIONEN

COMPOSITION OF DIBLOCK AND TRIBLOCK COPOLYMERS AND THE USE THEREOF IN THE PREVENTION OF TISSUE ADHESIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **04.08.2014   FR 1457562**

(43) Date de publication de la demande:
**14.06.2017   Bulletin 2017/24**

(73) Titulaires:
• **Université de Montpellier
34000 Montpellier (FR)**
• **Ecole Nationale Supérieure de Chimie de Montpellier
34090 Montpellier (FR)**
• **Centre National de la Recherche Scientifique
75016 Paris (FR)**
• **Centre Hospitalier Universitaire de Nîmes
30900 Nimes (FR)**

(72) Inventeurs:
• **COUDANE, Jean
F-34970 Lattes (FR)**

• **LEPRINCE, Salomé
F-66250 Saint Laurent De La Salanque (FR)**
• **GARRIC, Xavier
F-34070 Montpellier (FR)**
• **PANIAGUA, Cédric
F-34560 Poussan (FR)**
• **HUBERLANT, Stéphanie
F-34000 Montpellier (FR)**
• **LETOUZEY, Vincent
F-30000 Nimes (FR)**

(74) Mandataire: **Cabinet Becker et Associés
25, rue Louis le Grand
75002 Paris (FR)**

(56) Documents cités:
**EP-A1- 1 642 921     WO-A1-98/02171
US-A- 4 716 203**

**(Cont. page suivante)**

- **YANG D J ET AL: "Tissue anti-adhesion potential of biodegradable PELA electrospun membranes", ACTA BIOMATERIALIA, ELSEVIER, AMSTERDAM, NL, vol. 5, no. 7, 1 septembre 2009 (2009-09-01), pages 2467-2474, XP026500010, ISSN: 1742-7061, DOI: 10.1016/J.ACTBIO.2009.03.034 [extrait le 2009-04-01]**

(52) Classification Coopérative des Brevets (CPC):
(Cont.)

C-Sets
**A61L 31/06, C08L 67/025;**
**A61L 31/06, C08L 67/04**

## Description

L'invention a trait à une nouvelle composition de copolymères diblocs et/ou triblocs à base de blocs de polyester et de poly-oxyéthylène, et à son utilisation pour la fabrication d'un matériau anti-adhérentiel particulièrement adapté à la prévention des adhérences tissulaires et plus particulièrement des adhérences ou synéchies intra-utérines.

## Arrière-plan technologique

Les synéchies ou adhérences intra-utérines, également connues, dans leur degré le plus sévère, sous le nom de syndrome d'Asherman, consistent en un accolement partiel ou total des parois du col de l'utérus et/ou du corps utérin. Elles s'accompagnent souvent de douleurs abdominales chroniques et de dysménorrhées, et peuvent conduire à une infertilité mécanique par défaut de nidation. On admet aujourd'hui que les synéchies intra-utérines sont le plus souvent dues à un geste opératoire réalisé dans la cavité utérine, tel qu'une césarienne, une résection endométriale, un curetage suite à un accouchement ou une fausse couche, etc.

Une fois diagnostiquées, les synéchies intra-utérines sont généralement traitées par hystéroscopie opératoire, en utilisant des micro-ciseaux introduits dans la cavité utérine pour sectionner les ponts fibreux synéchiques. Le taux de reformation de synéchie reste cependant relativement élevé, et peut même atteindre plus de 40% en cas de synéchies sévères.

Actuellement, la prévention des synéchies n'est pas ou peu développée. Une solution consisterait en effet à mettre en place une barrière physique dans la cavité utérine des patientes à risque, afin de prévenir les accolements de tissus. Cependant, les produits développés jusqu'à maintenant ne donnent pas satisfaction.

Ainsi, des gels à base d'acide hyaluronique et/ou de polyéthylène glycol ont été développés (Hyalobarrier ® de Nordic Pharma, SprayShield® de Covidien), pour être déposés sur la paroi utérine à la fin d'une intervention chirurgicale. Cependant, du fait notamment de leur forme galénique en gel, ces produits sont éliminés très rapidement, et ont un temps de séjour dans l'utérus inférieur en moyenne à 24 heures. Or, on sait que les synéchies se développent dans un délai de 1 à 8 jours après la survenue d'un traumatisme utérin. L'élimination trop rapide de ces gels les empêche donc d'agir de manière satisfaisante pour prévenir la formation de ponts fibreux synéchiques.

Un film composé d'acide hyaluronique de synthèse, associé à un support de carboxyméthylcellulose/glycérol a également été développé (Seprafilm® de Genzyme). Si ce type de film est couramment utilisé pour prévenir les adhérences post-opératoires en chirurgie abdominale, il ne donne pas satisfaction pour les synéchies intra-utérines. En effet, la morphologie et les propriétés mécaniques du film font qu'il ne peut pas ou difficilement être introduit dans la cavité utérine depuis le col utérin.

On connait également du document US 7,202,281 une composition comprenant des copolymères à base d'acide poly-lactique (PLA) et de polyéthylène glycol (PEG) présentant un poids moléculaire de 6 000, utilisée pour former un film antiadhérentiel destiné à prévenir les synéchies notamment au niveau abdominal. Un tel film ne donne cependant pas satisfaction dans le cadre de la prévention des synéchies intra-utérines.

Il existe donc un réel besoin d'un produit pouvant être aisément positionné dans la cavité utérine et dont les propriétés physico-chimiques permettent de prévenir la formation de synéchies, notamment chez les patientes ayant subi une intervention chirurgicale dans la cavité utérine.

## Résumé de l'invention

Dans ce contexte, les inventeurs ont découvert que l'utilisation de copolymères à base de blocs de polyesters, tels que de l'acide poly-lactique (PLA), et de blocs de polyéthylène glycol (PEG) de haut poids moléculaire, permet de réaliser un matériau combinant des propriétés anti-adhérentielles de surface, de gonflement et de résorption particulièrement adaptées à une utilisation dans la cavité utérine pour y prévenir la formation de synéchies. Les inventeurs ont ainsi développé un dispositif médical à partir d'un tel matériau, qui sous forme « sèche » a des dimensions permettant une mise en place aisée depuis le col utérin, et qui une fois dans la cavité utérine s'humidifie et se déploie pour épouser les contours de la cavité utérine. En effet, le matériau selon l'invention présente un taux de gonflement en milieu aqueux lui permettant d'augmenter jusqu'à environ dix fois son volume par rapport à la forme « sèche ». En outre, le temps de désintégration de ce matériau est généralement compris entre 5 et 20 jours, ce qui permet non seulement de garantir un temps de séjour du dispositif médical suffisant dans la cavité utérine pour prévenir la formation de ponts fibreux synéchiques, mais également d'assurer son élimination naturellement, en particulier lors du cycle menstruel suivant.

L'invention a donc pour objet une composition à base de copolymères comprenant au moins un copolymère blocs A et B, dans lequel :

- le bloc A est un polyester,
- le bloc B est un poly-oxyéthylène (PEG),

- la masse moléculaire en poids des blocs B est supérieure ou égale à 50 kDa, et
- le ratio molaire motif éthylène oxyde/motif ester est de 0,5 à 5.

Avantageusement, la masse moléculaire des blocs B dans le copolymère est comprise entre environ 50 kDa et environ 300 kDa. Dans un mode de réalisation particulier, la masse moléculaire du PEG est comprise entre environ 75 kDa et environ 150 kDa, préférentiellement entre environ 80 et environ 125 kDa, plus préférentiellement entre environ 90 et environ 115 kDa, de manière encore préférée entre environ 100 kDa et environ 110 kDa.

Préférentiellement, la composition selon l'invention n'est constituée que de copolymères blocs A et B. Dans un mode de réalisation particulier, la composition comprend des copolymères triblocs ABA. Dans un autre mode de réalisation, la composition n'est constituée que de copolymères triblocs ABA.

L'invention a également pour objet un matériau anti-adhérentiel obtenu par mise en forme d'une telle composition à base de copolymères. Le matériau selon l'invention est tout particulièrement adapté à un usage médical notamment pour la prévention des adhérences tissulaires, et plus spécifiquement pour la prévention des synéchies intra-utérines. Le matériau peut notamment avoir la forme d'un film ou d'un tube.

L'invention a aussi pour objet un dispositif médical destiné à la prévention des synéchies intra-utérines comprenant ledit matériau anti-adhérentiel. Avantageusement, le dispositif médical comprend en outre des moyens d'insertion dudit matériau aptes à introduire et libérer ledit matériau dans la cavité utérine d'une patiente.

De même, l'invention a pour objet l'utilisation d'une telle composition, d'un tel matériau, et/ou d'un tel dispositif médical pour la prévention des adhérences tissulaires, et notamment des synéchies intra-utérines chez les patientes à risque. Par exemple, le matériau selon l'invention peut être utilisé de manière systématique chez les patientes venant de subir une césarienne, une interruption volontaire de grossesse ou tout geste chirurgical intra-utérin susceptible de provoquer des synéchies, tel qu'une résection bilatérale de fibromes, notamment par myomectomie.

L'invention a également pour objet une méthode de prévention des synéchies intra-utérines chez une patiente à risque, selon laquelle on introduit dans la cavité utérine de ladite patiente un matériau anti-adhérentiel réalisé à partir d'une composition à base de copolymères comprenant au moins un copolymère blocs A et B, dans lequel

- le bloc A est un polyester,
- le bloc B est un poly-oxyéthylène (PEG),
- la masse moléculaire des blocs B est supérieure ou égale à 50 kDa, et
- le ratio molaire motif éthylène oxyde/motif ester est de 0,5 à 5.

## Brève description des figures

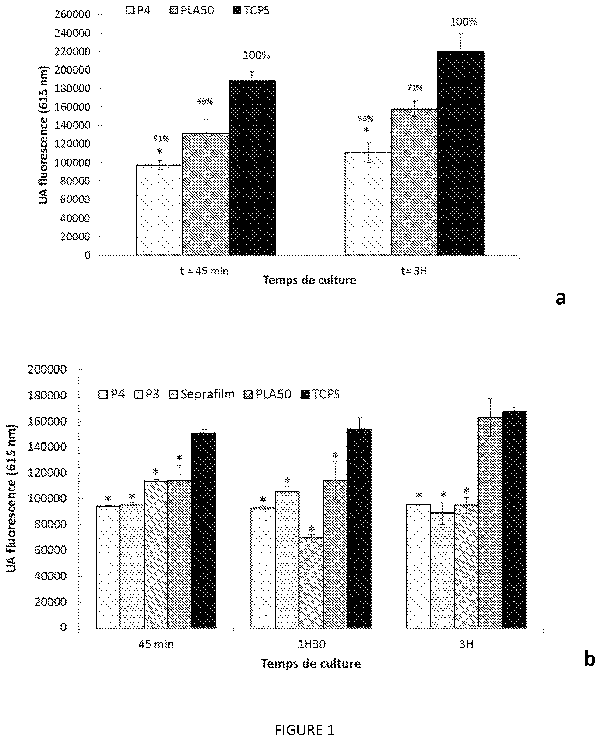

La **figure 1** montre les résultats obtenus lors d'un test d'adhésion cellulaire réalisé sur un matériau selon l'invention comprenant des copolymères triblocs PLA - PEG 95 kDa - PLA, avec un ratio molaire éthylène oxyde/acide lactique (EO/LA) de 3 (ci-après copolymère P4) et sur deux contrôles (TCPS et $PLA_{50}$). Les valeurs de fluorescence (UA 615 nm) reflètent la quantité de cellules ayant adhéré sur le film à t = 45 minutes et t = 3 heures. Les cellules utilisées sont des lignées fibroblastiques murines (L929) couramment utilisés pour l'évaluation in vitro des dispositifs médicaux (figure 1a). La figure 1b présente les résultats comparatifs obtenus lors d'un test d'adhésion cellulaire réalisé sur deux matériaux selon l'invention, à savoir le copolymère P4 et le copolymère triblocs PLA - PEG 95 kDa - PLA avec un ratio molaire EO/LA de 1 (ci-après copolymère P3), ainsi que sur un matériau anti-adhérentiel commercial Seprafilm et deux contrôles (TCPS et $PLA_{50}$). Les valeurs de fluorescence (UA 615 nm) reflètent la quantité de cellules ayant adhéré sur le film à t = 45 minutes, t = 1h30 heures et t=3 heures. Les cellules utilisées sont des cellules endométriales humaines (figure 1b);

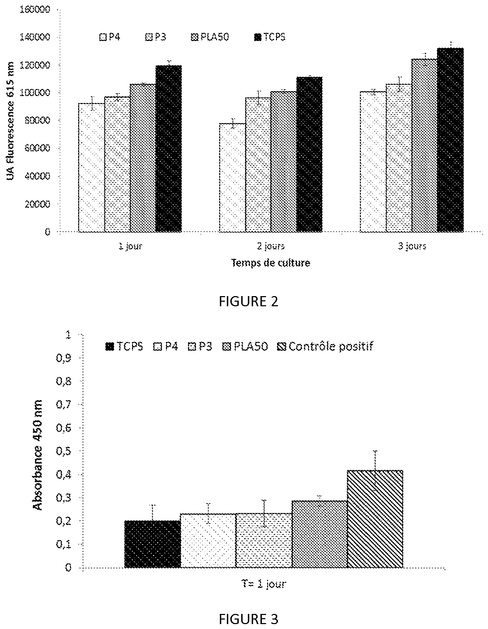

La **figure 2** montre les résultats obtenus lors d'un test de prolifération cellulaire réalisé sur un matériau selon l'invention réalisé à partir du copolymère P4, ainsi que sur un second matériau selon l'invention comprenant des copolymères triblocs PLA - PEG 95 kDa - PLA, avec un ratio molaire EO/LA de 1 (ci-après copolymère P3) et sur deux contrôles (TCPS et $PLA_{50}$). Les valeurs de fluorescence reflètent la quantité de cellules ayant proliféré sur les films à 1, 2 et 3 jours. Les cellules utilisées sont des lignées fibroblastiques murines (L929) couramment utilisés pour l'évaluation in vitro des dispositifs médicaux;

La **figure 3** montre les résultats obtenus lors d'un test de cytotoxicité réalisé sur un matériau à base de copolymères P4, un matériau à base de copolymères P3 et sur trois contrôles (TCPS, contrôle positif et $PLA_{50}$) en contact direct avec des cellules endométriales humaines. Les valeurs d'absorbance à 24 heures reflètent le taux de lyse cellulaire : Test de cytotoxicité. Copolymère P4 et copolymère P3 versus Contrôle positif P < 0,05 (test t-Student) ;

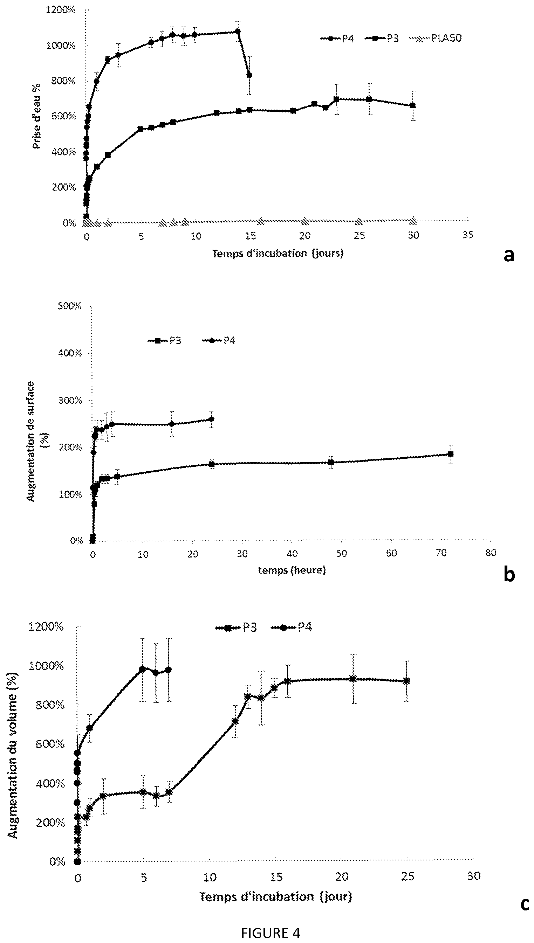

La **figure 4** (figures 4a, 4b et 4c) montre les propriétés de gonflement d'un matériau selon l'invention à base de copolymères P4, de copolymères P3, ou de PLA50 mis en forme par pressage à chaud. La figure 4a montre le pourcentage de prise d'eau desdits matériaux dans le temps, la figure 4b montre le pourcentage d'augmentation de surface correspondant, et la figure 4c montre le pourcentage d'augmentation du volume correspondant ;

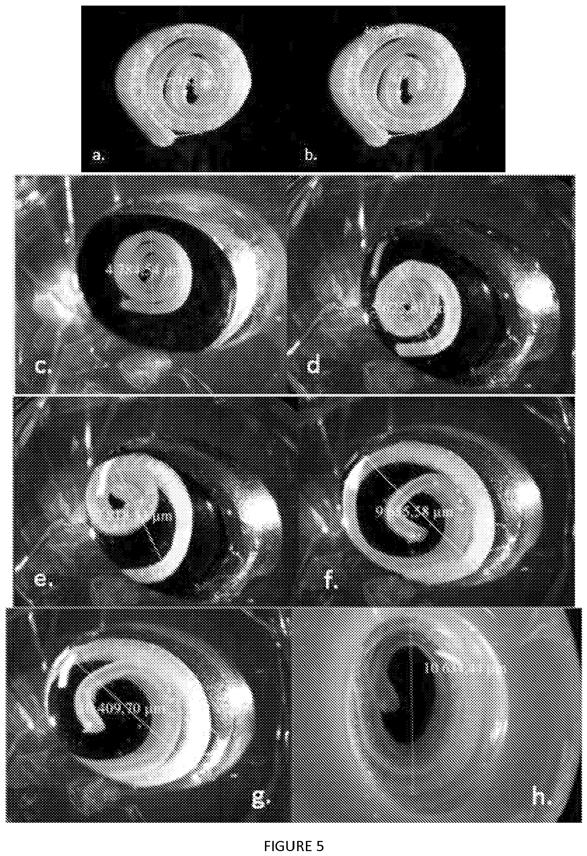

La **figure 5** montre la capacité de déploiement d'une bandelette de film réalisé à partir d'une composition selon l'invention à base de copolymères P4, enroulée avant immersion dans de l'eau (figure 5a et 5b) et à différents temps d'immersion, de t = 2 minutes à t = 24 heures (figures 5c - 5h). Les photos permettent de visualiser une augmentation de la longueur et de l'épaisseur du film : 5a. Diamètre totale de la bandelette enroulée (4 724 $\mu$m), 5b. Epaisseur de la membrane (463 $\mu$m). 5c. longueur à t=0 (4 784$\mu$m); 5d. longueur à t= 2 min (5 625$\mu$m) ; 5e. longueur à t= 7 min (7 818 $\mu$m) ; 5f. longueur à t= 15 min (9 655 $\mu$m) ; 5g. longueur à t= 45 min (10 409 $\mu$m) ; 5h. longueur à t= 24H (10 634 $\mu$m) ;

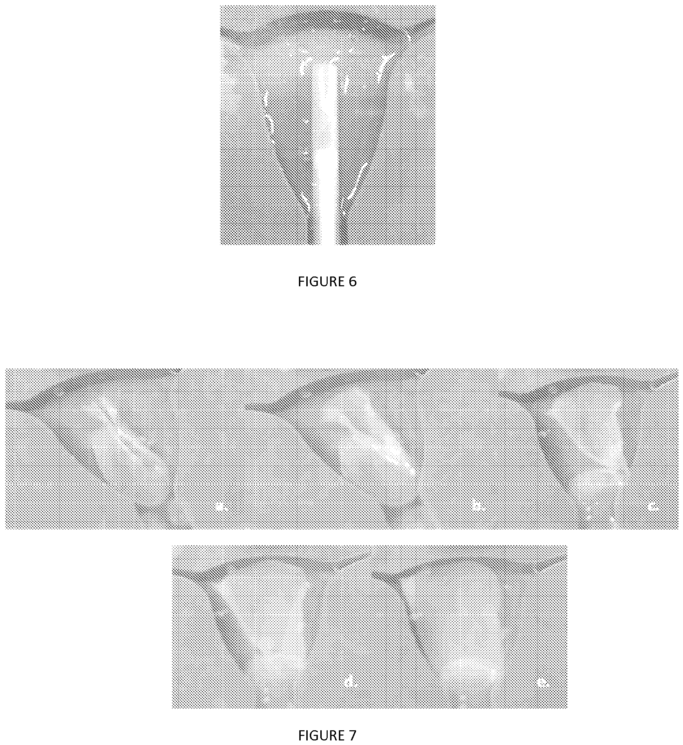

La **figure 6** est une photo d'un applicateur de stérilet dans lequel a été inséré un film selon l'invention (film P4), réalisé à partir d'une composition de copolymères P4, lors de son insertion dans un modèle mimant une cavité utérine remplie d'eau ;

La **figure 7** montre le déploiement progressif du film P4 libéré dans le modèle de cavité de la figure 6, à t=0 minute (figure 7a), t=1 minute (figure 7b), t=3 minutes (figure 7c), t=5 minutes (figure 7d) et t=8 minutes (figure 7e) ;

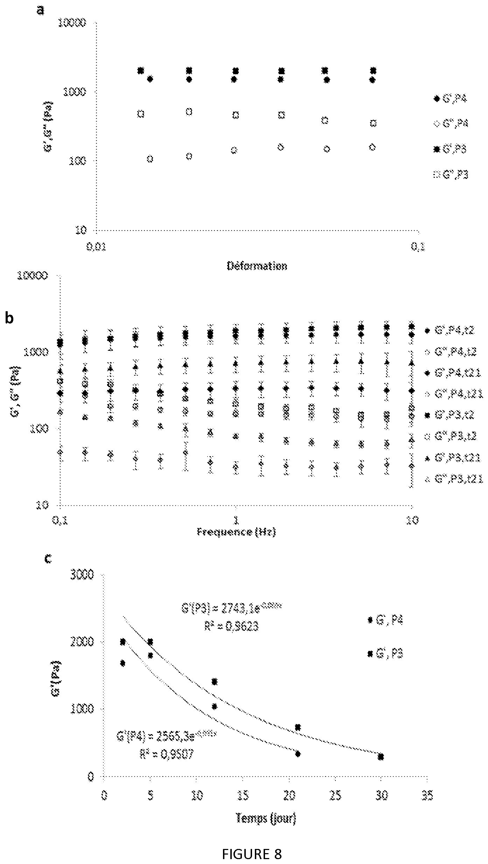

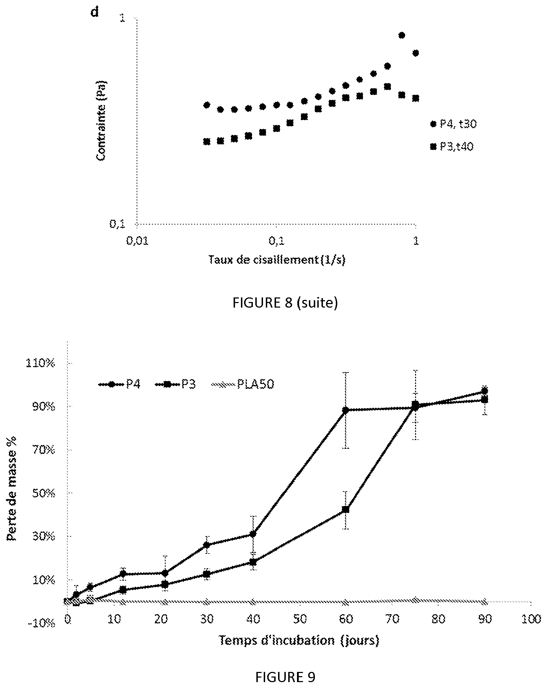

La **figure 8** montre les propriétés rhéologiques d'un film de copolymères P4 et d'un film de copolymères P3 lorsqu'ils sont immergés dans du PBS à 37°C sous agitation mécanique. La figure 8a montre le module élastique G' et le module visqueux G" des films de copolymères P3 et P4 après 5 minutes d'immersion, lorsqu'on leur applique une gamme de déformation à fréquence fixe (1Hz). La figure 8b montre la variation du module élastique (G') et du module visqueux (G") en fonction de la fréquence (à 0,05% de déformation) pour les copolymères P4 et P3 après 2 jours et 21 jours d'immersion. La figure 8c montre le module élastique (G') à différents temps d'incubation pour les copolymères P4 et P3. La figure 8d montre la contrainte des copolymères P4 après 30 jours d'incubation et la contrainte des copolymères P3 après 40 jours d'incubation en fonction du taux de cisaillement ;

La **figure 9** montre le pourcentage de perte de masse de deux films réalisés à partir de matériaux selon l'invention à base de copolymères P4, à base de copolymères P3 et d'un film de PLA50, lorsqu'ils sont mis à incuber en milieu aqueux. La figure montre la perte de masse progressive des matériaux au cours du temps, avec une solubilisation complète des films de copolymères P3 et P4 après 90 jours d'incubation ;

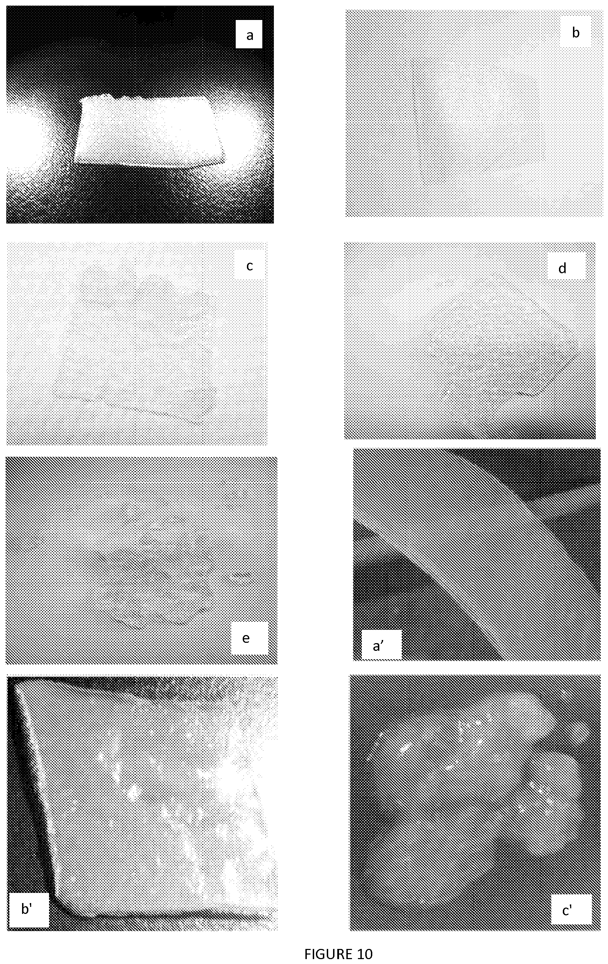

La **figure 10** montre la dégradation en milieu aqueux d'un film réalisé à partir d'un matériau selon l'invention à base de copolymères P4 à différents temps t = 24 heures (figure 10a), t = 5 jours (figure 10b), t = 12 jours (figure 10c), t = 22 jours (figure 10d) et t = 40 jours (figure 10e), ainsi que la dégradation d'un matériau à base de copolymères P3 à t = 24 heures (figure 10a'), t = 30 jours (figure 10b') et t = 60 jours (figure 10c'). Les photos des films montrent une désintégration progressive du film, jusqu'à sa désintégration quasi-totale ;

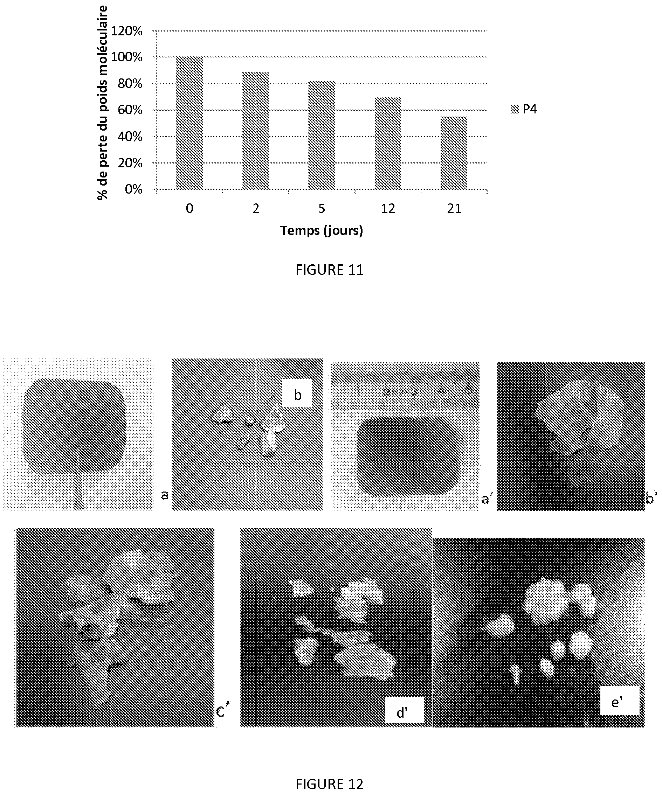

La **figure 11** montre la dégradation en milieu aqueux d'un film réalisé à partir d'un matériau selon l'invention à base de copolymères P4 suivie par Chromatographie d'Exclusion Stérique ;

La **figure 12** montre la dégradation *in vivo* d'un film réalisé à partir d'un matériau selon l'invention à base de copolymères P4 à différents temps t = 0 (figure 12a) et t = 2 jours (figure 12b), ainsi que la dégradation *in vivo* d'un film réalisé à partir d'un matériau selon l'invention à base de copolymères P3 à différents temps t = 0 (figure 12a'), t = 12 heures (figure 12b'), t = 2 jours (figure 12c'), t = 5 jours (figure 12d') et t = 12 jours (figure 12e') ;

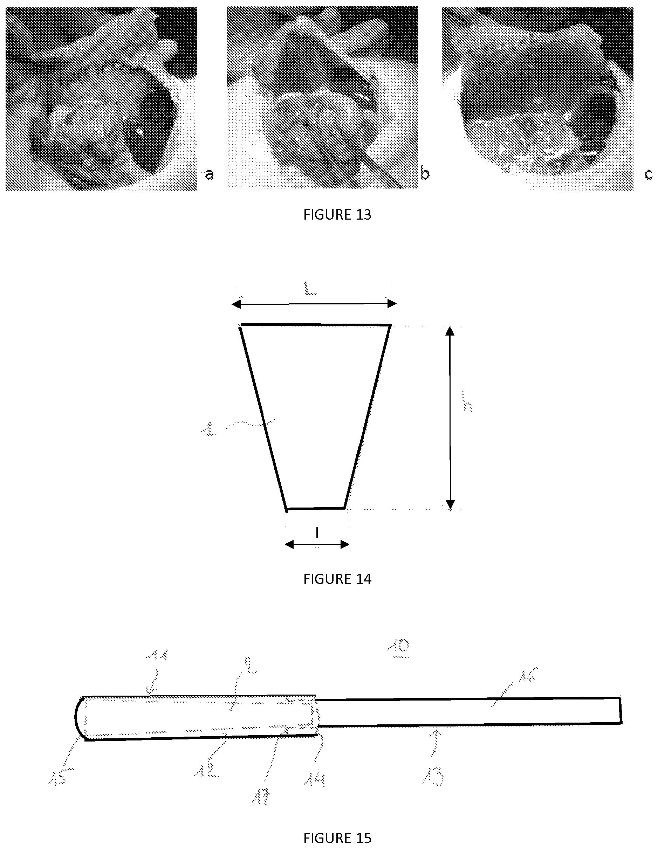

La **figure 13** montre l'état de la cavité abdominale d'une ratte 12 jours après un défect péritonéal et une abrasion de la séreuse caecale qui ont pour objectif de former une adhésion entre le péritoine et le caecum. La figure 13a montre l'adhésion du caecum et du péritoine 12 jours après la chirurgie sans qu'aucun traitement ou agent anti-adhérentiel ne soit appliqué (ratte témoin). La figure 13b montre l'état de la cavité abdominale dépourvue d'adhésion 12 jours après la chirurgie avec l'application d'un film réalisé selon l'invention à base de copolymères P4. La figure 13c montre l'état de la cavité abdominale dépourvue d'adhésion 12 jours après la chirurgie avec l'application d'un film réalisé selon l'invention à base de copolymères P3 ;

La **figure 14** est une représentation schématique d'un film anti-adhérentiel selon l'invention en forme de trapèze, particulièrement adapté à une utilisation dans une cavité utérine humaine, car apte à en épouser les contours après gonflement et déploiement ;

La **figure 15** est une représentation schématique en coupe longitudinale d'un exemple de réalisation du dispositif médical selon l'invention comprenant des moyens d'insertion du matériau anti-adhérentiel dans une cavité utérine.

### Description détaillée

Les inventeurs ont mis au point une composition comprenant des copolymères blocs à base de blocs de polyesters et de blocs de poly-oxyéthylène (PEG) de haut poids moléculaire présentant des propriétés mécaniques et chimiques particulièrement adaptées à une utilisation dans le domaine médical, et en particulier pour la prévention des synéchies intra-utérines. En effet, les propriétés de gonflement et déploiement de la composition, combinées à ses propriétés anti-adhérentielles et de dégradation en quelques jours, font qu'il est possible de l'utiliser de manière temporaire dans la cavité intra-utérine pour prévenir de manière fiable les adhérences des parois utérines.

*Composition*

La présente invention a pour objet une composition à base de copolymères comprenant au moins un copolymère blocs A et B, où A est un polyester et B est du poly-oxyéthylène (PEG), et dans laquelle la masse moléculaire des blocs B dans le copolymère est supérieure ou égale à 50 kDa et le ratio molaire motif éthylène oxyde/motif ester est compris entre 0,5 et 5.

Dans le contexte de l'invention, l'expression « compris entre x et y » signifie que les valeurs x et y sont incluses.

Selon l'invention le terme « polyester » désigne tout polymère dont les motifs de répétition de la chaîne principale contiennent la fonction ester et qui peut être utilisé dans le domaine médical. Notamment, les polyesters s'entendent des polyesters aliphatiques tels que poly (acide lactique) (PLA), le poly (acide glycolique), la poly-caprolactone (PCL), la poly-butyrolactone (PBL), les poly-hydroxyalcanoates (PHA), et leurs copolymères.

Dans un mode de réalisation préféré, le polyester (bloc A) est choisi parmi le poly(acide lactique) (PLA), le poly(acide glycolique), la poly-caprolactone (PCL) et leurs copolymères.

Préférentiellement, le polyester présent dans la composition est sous une forme non réticulée.

Le poly (acide lactique) peut être le poly (acide L-lactique), le poly (acide D-lactique) ou le poly (acide D,L lactique). Avantageusement, on utilise le poly (acide D,L lactique) (PDLLA). Dans ce cas, le polymère comporte préférentiellement au moins 50% d'acide L-lactique, et notamment au moins 60%, 70%, 75%, 80%, 85%, 90%, 95% ou 99%. En effet, en modifiant le pourcentage d'acide L-lactique par rapport à l'acide D-lactique, il est possible de jouer sur la vitesse de dégradation du copolymère blocs A et B. Une augmentation du taux d'acide L-lactique permet de ralentir la vitesse de dégradation du copolymère. Dans certains modes de réalisation de l'invention, la composition comprend 100% de PLLA en tant que blocs A.

Selon l'invention, le PEG utilisé présente un haut poids moléculaire, de manière à ce que le poids moléculaire total du PEG dans le copolymère soit supérieur ou égal à 50 kDa. En effet, les inventeurs ont découvert qu'un copolymère dans lequel le PEG a un poids moléculaire supérieur ou égal à 50 kDa, préférentiellement supérieur à 75 kDa, et de manière préférée supérieur ou égale à environ 100 kDa, permet de conférer à la composition des propriétés filmogènes particulièrement intéressantes dans le cadre d'une utilisation médicale sous forme de film ou membrane anti-adhérentielle. Par « propriétés filmogènes », on entend que la composition est capable de former un film homogène et continu. Plus particulièrement, ces propriétés filmogènes rendent la composition particulièrement stable et homogène dans le temps et conduisent à la formation d'un film présentant une bonne tenue en milieu aqueux.

Dans le contexte de l'invention, on utilise indifféremment les termes « masse moléculaire » et « poids moléculaire » pour désigner, sauf mention contraire, la masse moléculaire moyenne en poids (Mw). Selon l'invention, la Mw est déterminée par chromatographie d'exclusion stérique réalisée dans du diméthylformamide en tant que solvant d'analyse, en utilisant une gamme étalon de Poly (éthylène glycol).

Avantageusement, la masse moléculaire totale du PEG dans le copolymère est comprise entre environ 50 et environ 300 kDa. Par exemple, les blocs PEG ont une masse moléculaire d'environ 50 kDa, 75 kDa, 100 kDa, 125 kDa, 150 kDa, 200 kDa, 225 kDa, 250 kDa, 275 kDa ou 300 kDa. Dans un mode de réalisation particulier, les blocs PEG utilisés ont une masse moléculaire comprise entre environ 75 kDa et environ 150 kDa, préférentiellement entre environ 80 et environ 125 kDa, plus préférentiellement entre environ 90 et environ 115 kDa, de manière encore préférée entre environ 100 kDa et environ 110 kDa. Dans un mode particulier, les blocs PEG utilisés ont une masse moléculaire comprise entre 95 et 105 kDa.

Selon l'invention, les blocs PEG utilisés ont avantageusement une viscosité inhérente comprise entre 0,04 mg/ml et 0,6 mg/ml, préférentiellement entre 0,08 mg/ml et 0,5 mg/ml, et de manière encore préférée entre 0,1 mg/ml et 0,3 mg/ml lorsqu'elle est mesurée par un viscosimètre capillaire de type Ubbelohde à une concentration de 1 g/l, à 25°C dans le chloroforme.

Avantageusement, la composition à base de copolymères selon l'invention comprend des copolymères diblocs AB, ou triblocs ABA ou BAB, ou des mélanges de ceux-ci, notamment [ABA et BAB], [AB et ABA], [AB et BAB], [ABA et BAB et AB]. Préférentiellement, la composition ne comprend que des copolymères blocs A et B. Dans un mode de réalisation particulier, la composition selon l'invention ne comprend que des copolymères triblocs ABA ou BAB, et préférentiellement que des copolymères triblocs ABA. Selon l'invention, dans une composition de copolymères AB et/ou ABA chaque bloc PEG a une masse moléculaire supérieure ou égale à 50 kDa et avantageusement comprise entre environ 50 et environ 300 kDa, préférentiellement entre environ 75 kDa et environ 150 kDa, de manière préférée entre environ 80 et environ 125 kDa, plus préférentiellement entre environ 90 et environ 115 kDa, de manière encore préférée entre environ 100 kDa et environ 110 kDa, voir entre 95 et 105 kDa, tandis que dans une composition comprenant des copolymères BAB, la somme des masses moléculaires des blocs PEG dans ledit copolymère est supérieure ou égale à 50 kDa et avantageusement comprise entre environ 50 et environ 300 kDa, préférentiellement entre environ 75 kDa et environ 150 kDa, de manière préférée entre environ 80 et environ 125 kDa, plus préférentiellement entre environ 90 et environ 115 kDa, de manière encore préférée entre environ 100 kDa et environ 110 kDa, voir entre 95 et 105 kDa.

Dans le contexte de l'invention, le ratio molaire représente le ratio molaire en chacun des motifs de répétition (ou unités) des blocs A et B. Le bloc B étant du PEG, les motifs de répétition sont des éthylènes oxyde (« motif éthylène oxyde » ou EO), tandis que les motifs de répétition du bloc A (« motif ester ») sont des acides carboxyliques tels que des motifs d'acides lactiques. Selon l'invention, le ratio molaire EO/motif ester dans les copolymères de la composition est compris entre 0,5 et 5, et de manière préférée entre environ 1 et 3. Le ratio molaire est mesuré à partir du spectre RMN (Résonance Magnétique Nucléaire) du proton dans le chloroforme deutéré du copolymère dans lequel on peut identifier les déplacements chimiques des pics caractéristiques des copolymères PLA-PEG-PLA : CH (PLA) : 5.1 ppm; $CH_2$(PEG) : 3.5 ppm; $CH_3$(PLA) : 1.5 ppm). Les compositions dont les copolymères ont un ratio molaire EO/motif ester, et notamment motif éthylène oxyde/ motif acide lactique (EO/AL), compris entre environ 1 et 3 présentent des propriétés de gonflement et de déploiement telles que le volume augmente jusqu'à dix fois en milieu aqueux, ce qui rend ces compositions particulièrement adaptées à une utilisation médicale pour la prévention des synéchies intra-utérines. On constate que les compositions présentant un ratio molaire EO/AL d'environ 3 se dégradent dans un délai plus court, de l'ordre de 3 à 5 jours, dans un milieu aqueux tel que du tampon phosphate salin PBS, que les compositions comprenant un ratio molaire EO/AL d'environ 1, qui elles se dégradent dans un délai d'environ 5 à 20 jours.

Dans le contexte de l'invention, le terme « environ » signifie la valeur considérée plus ou moins 10%.

Selon l'invention, un « milieu aqueux » fait référence à un milieu présentant une osmolarité similaire à l'osmolarité des fluides biologiques. On utilise communément en tant que milieu aqueux, du tampon phosphate salin (PBS) considéré comme représentatif des fluides biologiques.

Dans un mode de réalisation particulier, la composition est constituée de copolymères triblocs ABA, où le bloc A est du PDLLA et le bloc B du PEG de poids moléculaire environ 100 kDa, dans laquelle le ratio molaire EO/AL est égal à environ 1.

Dans un autre mode de réalisation particulier, la composition est constituée de copolymères triblocs ABA, où le bloc A est du PDLLA et le bloc B du PEG de poids moléculaire 100 kDa, dans laquelle le ratio molaire EO/AL est égal à environ 3.

La composition selon l'invention peut être obtenue par toute méthode de synthèse de copolymères blocs connue de l'homme du métier. Par exemple, un copolymère de type ABA peut être obtenu par polymérisation en chaine depuis les extrémités du bloc B. Typiquement, on réalise une polymérisation par ouverture de cycle de lactide amorcée par les hydroxyles terminaux du bloc PEG en présence d'un catalyseur tel que l'octanoate d'étain. Cette polymérisation peut être réalisée en l'absence ou en présence de solvants. Un copolymère de type BAB peut par exemple être préparé par couplage de méthoxy-PEG sur une chaîne PLA dont les deux extrémités de chaîne sont des fonctions acide-carboxylique. Un tel PLA « difonctionnalisé » est obtenu par exemple par traitement d'une chaîne de PLA par de l'anhydride succinique ou adipique.

Dans un mode de réalisation particulier, la composition selon l'invention comprend des copolymères blocs A et B, où les blocs B (PEG à haut poids moléculaire) sont obtenus à partir de plusieurs PEG de plus faible poids moléculaires. Par exemple, la composition comprend du PEG 100 kDa réalisé avec des blocs de PEG plus courts (par exemple entre 5 et 25 kDa), reliés entre eux par des liaisons dégradables. Lors de la dégradation des copolymères, les blocs PEG 100 kDa se dégradent alors en sous-unités de 5 à 25 kDa, qui peuvent passer la filtration glomérulaire et être ainsi éliminées par les urines. Ce mode de réalisation est particulièrement intéressant lorsque l'on souhaite utiliser la composition selon l'invention pour la prévention d'adhérences tissulaires autres que les synéchies intra-utérines.

*Matériau anti-adhérentiel*

L'invention a également pour objet un matériau anti-adhérentiel obtenu par mise en forme de la composition à base de copolymères blocs A et B décrite ci-dessus.

La mise en forme du matériau peut se faire par tout moyen connu de l'homme du métier, et notamment par extrusion, évaporation par solvant en utilisant par exemple du dichlorométhane, pressage à chaud, moulage ou impression 3D.

D'une manière générale, l'épaisseur du matériau obtenu dépend de la quantité de composition utilisée et de la surface du support ou du moule utilisé pour la mise en forme.

Le matériau selon l'invention peut ainsi prendre la forme d'un film, d'un tube, d'une structure poreuse, telle qu'une matrice 2D ou 3D de gel ou hydrogel poreux, etc.

Un film s'entend d'un matériau en deux dimensions, résultant de l'évaporation sur une surface plane du solvant ayant solubilisé le copolymère de la composition selon l'invention. L'épaisseur d'un tel film est avantageusement comprise entre quelques microns et plusieurs centaines de microns, et notamment entre 10 $\mu$m et 500 $\mu$m. Dans un mode de réalisation particulier, le film a une épaisseur comprise entre 100 $\mu$m et 300 $\mu$m environ. L'épaisseur s'entend « sèche », en ce sens qu'elle est mesurée (par exemple par microscopie optique) en conditions anhydres, après mise en forme et évaporation totale du solvant ayant servi à solubiliser le copolymère.

Les dimensions du film peuvent être adaptées selon les besoins, notamment en découpant un film de plus grandes dimensions aux dimensions souhaitées.

Les films peuvent être repliés pour former des tubes, ou des manchons, maintenus fermés au besoin par suture ou collage. Les tubes peuvent également être obtenus directement par mise en forme autour d'un cylindre ou par extrusion.

Dans le contexte de l'invention, un tube désigne un objet cylindrique en trois dimensions, creux, dont les parois sont formées d'un film de copolymères comprenant au moins un copolymère blocs A et B selon l'invention. Préférentiellement, le diamètre d'un tel tube est de plusieurs centaines de microns, et notamment compris entre 300 $\mu$m et 3000 $\mu$m. Dans un mode de réalisation particulier, le tube présente une épaisseur de parois d'environ 300 $\mu$m et un diamètre d'environ 900 $\mu$m.

Dans un mode de réalisation particulier, le matériau est obtenu par mise en forme de la composition sur un support destiné à former une partie dudit matériau. Par exemple, la composition est séchée sur un textile tissé ou tricoté constitué d'un autre polymère, l'ensemble formant ainsi un matériau mixte.

Avantageusement, le matériau ne comprend que les constituants de la composition à base de copolymères selon l'invention, et éventuellement des traces de solvant. Par exemple, le matériau ne comprend que des copolymères de PLA et de PEG de haut poids moléculaire (supérieur ou égal à 50 kDa). Dans un exemple particulier, le matériau ne comprend que des copolymères de PLA et de PEG, dans lesquels le PEG a un poids moléculaire d'environ 100 kDa.

Dans certains cas, le matériau peut comporter, outre les constituants de la composition selon l'invention, un additif ou principe actif additionnel, tel qu'une molécule anti-adhérentielle ou une molécule à visée thérapeutique telle qu'un antibiotique. Cet additif ou principe actif peut par exemple être ajouté à la composition à base de copolymères avant ou pendant la mise en forme du matériau, de manière à être dispersé dans la matrice polymérique du matériau. Autrement, il est possible d'imprégner ou recouvrir le matériau avec ce principe actif après la mise en forme. Préférentiellement, le principe actif est apte à diffuser vers l'extérieur du matériau quand il est dans un milieu aqueux.

Le matériau selon l'invention présente des propriétés anti-adhérentielles, de résorption et de gonflement particulièrement adaptées à une utilisation médicale pour la prévention des adhérences tissulaires et notamment des synéchies intra-utérines.

Par propriétés anti-adhérentielles, on entend que le matériau selon l'invention est apte à prévenir ou à tout le moins limiter la prolifération cellulaire et l'adhésion cellulaire à sa surface, comparativement à un matériau ne présentant pas de telles propriétés. Les propriétés anti-adhérentielles peuvent être mesurées en évaluant au cours du temps à la surface du matériau, le taux d'adhésion cellulaire par mesure d'une fluorescence reflétant la quantité de cellules ayant adhéré à la surface du matériau. De manière similaire, on peut évaluer le taux de prolifération cellulaire également par mesure de fluorescence à la surface du matériau.

L'utilisation de PEG de haut poids moléculaire dans la composition utilisée pour former le matériau permet de lui conférer des propriétés anti-adhérentielles particulièrement intéressantes dans le cadre de la prévention des synéchies, et notamment des synéchies intra-utérines. Ainsi, un matériau obtenu par mise en forme d'une composition à base de copolymères triblocs ABA où A est du PDLLA et B du PEG 100.000 (c'est-à-dire de poids moléculaire environ 100 kDa) et dont le ratio molaire EO/AL est d'environ 1 ou d'environ 3 présente un taux d'adhésion cellulaire 2 fois plus faible que le taux obtenu avec des films adhérentiels de l'état de la technique.

Avantageusement, le matériau selon l'invention présente, dans un milieu aqueux présentant une pression osmotique identique à celle des fluides biologiques, un taux de gonflement compris entre environ 1 et 20, et de manière préférée compris entre environ 3 et 15. Préférentiellement, le matériau selon l'invention présente un taux de gonflement au moins égal à 4. Le taux de gonflement est mesuré de la manière suivante : on pèse une bandelette de matériau sec avant de l'immerger pendant 24 heures à 37°C en milieu salin (PBS 1X) sous agitation. Après 24 heures, l'excès de PBS est retiré avec du papier absorbant et la bandelette est à nouveau pesée. Le taux de gonflement correspond au rapport masse de la bandelette de matériau humide / masse de la bandelette de matériau sec. Le taux de gonflement du matériau est proportionnel à son pourcentage de prise d'eau, qui correspond au rapport [(masse de la bandelette de matériau humide - masse de la bandelette de matériau sec)/ masse de la bandelette de matériau sec] x 100.

Le gonflement du matériau s'accompagne d'une augmentation de surface et de volume (surface ou volume « hydraté(e) ») ce qui est particulièrement avantageux lors d'une utilisation médicale pour la prévention des synéchies intra-utérines, puisque cela favorise le déploiement du matériau dans la cavité utérine, en particulier pour en épouser les contours.

L'augmentation de surface est très rapide et peut notamment atteindre 200 à 300 % en l'espace de quelques minutes à quelques heures en milieu aqueux. On observe avantageusement un plateau autour de 300 à 400% après 4 à 5 heures en milieu aqueux. L'augmentation de surface s'accompagne d'une augmentation de volume du matériau, qui se mesure visuellement, dans les mêmes conditions que lors de la mesure du taux de gonflement, en modifiant uniquement les temps de séjour dans la solution saline. L'augmentation de surface correspond au rapport [(surface de la bandelette « hydratée » après un temps t d'immersion - surface de la bandelette « sèche ») / surface de la bandelette « sèche »] x 100.

Une caractéristique additionnelle particulièrement avantageuse du matériau selon l'invention est qu'il ne se dégrade pas rapidement en milieu aqueux, permettant un temps de séjour dans la cavité utérine compris entre 1 et 40 jours, préférentiellement entre 2 et 20 jours, et plus préférentiellement entre 3 et 15 jours, tout en conservant son intégrité pendant un temps compris au moins entre 1 et 15 jours. La dégradation du matériau est due à l'hydrolyse progressive des liaisons ester des blocs de polyester suivie d'une solubilisation des blocs contenant du PEG. La perte des propriétés mécaniques du matériau est directement liée à sa dégradation. La dégradation peut s'évaluer en mesurant dans le temps la diminution du poids moléculaire d'une bandelette de matériau, après immersion à 37°C en milieu salin (PBS 1X) sous agitation, par exemple par Chromatographie d'Exclusion Stérique. Il est également possible d'évaluer la diminution de la viscosité dynamique du matériau. On observe généralement une perte de viscosité de l'ordre de 30 à 50% après 7 jours d'immersion à 37°C en milieu salin (PBS 1X) sous agitation, et jusqu'à 70% après 15 jours.

On observe également une modification des propriétés mécaniques du matériau selon l'invention in vivo. Plus précisément, celui-ci passe de l'état de film à l'état de gel en quelques jours. Notamment, un copolymère triblocs ABA dans lequel A est du PLA et B du PEG de poids moléculaire environ 100 kDa, le passage de l'état film à l'état de gel survient au bout de 2 jours lorsque le ratio EO/LA est de 3, et au bout de 12 jours lorsque le ratio EO/LA est de 1. Ces modifications des propriétés mécaniques sont directement liées à la dégradation hydrolytique.

Ainsi, la composition selon l'invention présente une balance entre le taux de gonflement des polymères et leur vitesse de dégradation qui permet de garantir au matériau obtenu à partir d'une telle composition un temps de séjour dans la cavité utérine pendant une période suffisante, avantageusement comprise entre 3 et 15 jours environ, pendant laquelle les propriétés mécaniques et l'intégrité physique dudit matériau sont au moins partiellement préservées. Notamment, les propriétés anti-adhérentielles et le volume « hydraté » du matériau perdurent pendant un temps suffisant pour prévenir la formation de synéchies lorsque ledit matériau est mis en place dans la cavité utérine d'une patiente après un traumatisme de la paroi utérine. La solubilisation des blocs PEG et l'hydrolyse des blocs polyesters dans la cavité utérine est progressive, et est généralement suffisante après 10 à 15 jours pour permettre l'élimination du matériau, en particulier lors des menstruations qui suivent généralement la mise en place dudit matériau dans la cavité utérine.


Dispositif médical et utilisation


L'invention a également pour objet un dispositif médical destiné à la prévention des synéchies intra-utérines comprenant le matériau anti-adhérentiel selon l'invention.

Dans un mode de réalisation particulier, le dispositif médical comprend un film ou un tube obtenu par mise en forme d'une composition constituée de copolymères triblocs ABA où A est du PDLLA et B du PEG de poids moléculaire d'environ 100 kDa, dans laquelle le ratio EO/AL est d'environ 1.

Dans un autre mode de réalisation, le dispositif médical comprend un film ou un tube obtenu par mise en forme d'une composition constituée de copolymères triblocs ABA où A est du PDLLA et B du PEG de poids moléculaire d'environ 100 kDa, dans laquelle le ratio EO/AL est d'environ 3.

Avantageusement, le dispositif médical comporte un film en matériau selon l'invention présentant une forme en triangle ou en trapèze, afin d'épouser au mieux, une fois hydraté et déployé par gonflement, les contours internes de la cavité utérine dans laquelle il est destiné à être logé.

Le film peut par exemple avoir une épaisseur sèche de 300 à 500 microns environ.

Dans un mode de réalisation particulier, tel que représenté à la figure 14, le film 1 a une forme de trapèze de hauteur h comprise entre 1 et 2 cm environ, de plus grande largeur L comprise entre 1 et 1,5 cm environ, et de plus petite largeur 1 comprise entre 0,25 et 0,75 cm environ. Par exemple, le trapèze présente une hauteur d'environ 1,5 cm, une plus grande largeur L d'environ 1,25 cm et de plus petite largeur 1 d'environ 0,5 cm. Ces dimensions sont aisément adaptables par l'homme du métier, notamment dans les fourchettes ci-dessus, en fonction du type de patiente à traiter, selon qu'elle est primipare ou multipare, de son âge, des raisons faisant craindre la survenue de synéchies, etc.

Le dispositif selon l'invention comprend avantageusement des moyens d'insertion et de mise en place du matériau dans la cavité utérine.

Par exemple, comme cela est représenté à la figure 15, le dispositif 10 selon l'invention comprend un inserteur 11 cylindrique creux, dans l'alésage 12 duquel un film 2 ayant une forme en trapèze inversé est logé. Avantageusement, afin de réduire au maximum les dimensions de l'inserteur 11, le film est logé dans l'alésage 12 sous une forme compactée. Par exemple, le film est plié en accordéon et maintenu serré par les parois internes de l'alésage 12. Ce n'est qu'une fois libéré dans la cavité utérine que l'accordéon se déploie. Ce déploiement est par ailleurs favorisé par l'augmentation quasi concomitante de volume du film, dont les polymères se gonflent d'eau au contact du liquide intra-utérin.

Le dispositif médical 10 comporte avantageusement un poussoir 13 monté coulissant en translation à une extrémité distale 14 de l'inserteur, l'extrémité proximale 15 opposée étant l'extrémité par laquelle l'inserteur 11 est destiné à être introduit dans la cavité utérine. Le poussoir 13 est constitué d'une tige 16 qui, lorsqu'elle est poussée à l'intérieur de l'alésage 12 de l'inserteur 11, vers l'extrémité proximale 15, entraine en translation le film 2 vers l'extérieur de l'inserteur 11.

Avantageusement, le poussoir 13 comprend des moyens d'arrêt 17 à l'extrémité proximale de la tige 16, lesdits moyens d'arrêt 17 étant destinés à venir en butée contre la paroi de l'alésage 11 bordant l'extrémité proximale 15 de l'inserteur, afin d'informer la personne manipulant le dispositif 10 que le film 2 a été entièrement éjecté de l'inserteur et qu'il est en position dans la cavité utérine. Il suffit alors de retirer l'ensemble moyens d'insertion/inserteur par simple traction vers l'extérieur, le film 2 restant lui en position dans la cavité utérine.

Un tel système permet d'introduire et de mettre en place de manière fiable le matériau anti-adhérentiel selon l'invention. Par ailleurs, cette forme compactée permet de réduire encore les dimensions du matériau avant gonflement, ce qui facilite son introduction par le col utérin de la patiente.

Le dispositif médical selon l'invention peut avantageusement être utilisé de manière systématique chez les patientes venant de subir une césarienne, ou toute autre intervention chirurgicale au niveau de la cavité abdominaux-pelvienne. La forme compactée du matériau anti-adhérentiel et l'emploi d'un applicateur de faibles dimensions facilitent sa mise en place dans la cavité utérine souvent sensible de ces patientes. En outre, son élimination naturelle lors du cycle menstruel permet d'éviter aux patientes une intervention supplémentaire du personnel médical visant à retirer ledit dispositif.

L'invention va maintenant être illustrée à l'aide des exemples ci-dessous. Ceux-ci sont présentés à titre indicatif et nullement limitatif de l'invention.

## EXEMPLES

### 1- Synthèse des polymères triblocs ABA

*Matériel*

Poly (éthylène-oxyde) commercial (PEG) : Fournisseur Sigma Aldrich, N°CAS 25322-68-3. Le PEG commercial a été analysé au laboratoire par Chromatographie d'exclusion stérique (CES) afin de déterminer sa masse molaire moyenne en poids (Mw). L'analyse a été réalisée dans un solvant d'analyse (le diméthylformamide), et la Mw a été déterminée via une gamme étalon de Poly (éthylène glycol).

La masse molaire moyenne en poids Mw est de 95 000 Da et sa viscosité inhérente est de 0,16 ml/mg.

Le Lactide utilisé dans la composition peut être du D, L-Lactide, du D- Lactide, du D -Lactide ou bien l'association du D-Lactide et du D-Lactide. Les proportions de Lactide introduites lors de la synthèse dépendent du pourcentage de L ou de D-Lactide souhaité dans la composition du polymère final.

*Méthode*

Le tribloc ABA est synthétisé de la façon suivante :

Le PEG (Mw 95,000) et le lactide sont séchés sous vide à température ambiante pendant 24H. Le PEG et le lactide (la quantité de lactide dépend de la longueur du bloc A souhaitée) sont introduits dans un ballon de polymérisation en présence d'octanoate d'étain. 10 cycles successifs de vide ($10^{-3}$ bar) et d'argon sont ensuite réalisés. Le mélange est ensuite chauffé à 140°C et on procède de nouveau à 10 cycles successifs de vide et d'argon. Le mélange est remis à température ambiante, puis placé dans un bain de glace. Une fois cristallisé, le mélange réactionnel est mis sous vide dynamique pendant 30 min, puis scellé sous vide dynamique. Le mélange est ensuite placé à l'étuve sous rotation mécanique à 140°C pendant 3 jours. Le mélange est solubilisé dans du dichlorométhane et précipité dans un mélange éther/éthanol. Le précipité est récupéré puis séché sous vide pendant 24H.

*Caractérisation*

L'un des triblocs ABA présenté dans ce rapport est obtenu à partir de PEG (Mw 95,000 Da) et de D, L-Lactide. La composition finale du copolymère, dit copolymère P4, a été déterminée par Résonance Magnétique Nucléaire du proton RMN $^1$H et on en déduit un ratio molaire EO/LA de 3.

L'analyse RMN à deux dimensions (DOSY) indique que la synthèse a bien abouti à un tribloc ABA.

Le copolymère a également été analysé par Chromatographie d'Exclusion Stérique (CES) afin de déterminer sa masse molaire moyenne Mw et sa dispersité Ip.

Avec un solvant d'analyse tel que le Diméthylformamide et en utilisant une gamme étalon de Poly (éthylène glycol), on obtient une Mw de 123,000 Da et une dispersité voisine de 5.

De plus, l'Analyse Thermogravimétrique (ATG) a permis de déterminer la température de dégradation du copolymère P4, qui est de 256 °C.

2- Mise en forme du matériau anti-adhérentiel

La formation d'un film anti-adhérentiel à partir de la composition de copolymères triblocs est réalisée comme suit :

A. Par évaporation de solvant : le copolymère est dissous dans du dichlorométhane (50 mg/ml). La solution est déposée sur un support et le dichlorométhane s'évapore lentement pour former un film. L'épaisseur du film dépend du volume de solution de copolymères dissout utilisé et de la surface du support.

B. Par pressage à chaud : le copolymère est chauffé à 100°C pendant 10 min puis placé entre deux plateaux chauffants. On applique ensuite une pression (3 000 psi) entre les deux plateaux. L'épaisseur du film dépend de la quantité de copolymère utilisée et de la surface du support.

C. Par impression tri-dimensionnelle via la technologie FMD (Dépôt de Matière en Fusion) : le copolymère est préalablement extrudé (T° = 120°C) pour former un filament homogène. Le filament de copolymère est ensuite utilisé pour l'impression de l'objet modélisé en trois dimensions.

D'une manière générale, on désigne par film P3, film P4 et film PLA50 des films réalisés à partir de compositions de copolymères P3, P4 et PLA50 respectivement.

3- Evaluation des propriétés anti-adhérentielles et de la biocompatibilité du matériau anti-adhérentiel

L'objectif de cette étude est de mettre en évidence l'effet anti-adhérentiel et l'absence de cytotoxicité.

Pour l'ensemble des tests réalisés en culture cellulaire, les copolymères ont comme composition :

- Tribloc ABA obtenu à partir de PEG (Mw 95,000) et de D, L-Lactide. Le ratio molaire final EO/LA est de 3 (Déterminé par Résonance Magnétique Nucléaire du proton RMN $^1$H). Le polymère est appelé copolymère « P4 ».

*Mise en forme d'un film de copolymères P4 (ci-après film P4) :* par évaporation de solvant. Epaisseur de 300 $\mu$m (mesure obtenue au microscope optique).

- Tribloc ABA obtenu à partir de PEG (Mw 95,000) et de D, L-Lactide. Le ratio molaire final EO/LA est de 1 (déterminé par Résonance Magnétique Nucléaire du proton RMN $^1$H). Le copolymère est appelé copolymère « P3 ».

*Mise en forme d'un film de copolymères P3 (ci-après film P3) :* par évaporation de solvant. Epaisseur de 300 $\mu$m (mesure obtenue au microscope optique).

Les contrôles utilisés sont choisis parmi les contrôles suivants

- Contrôle positif : TCPS (puits) ;
- Contrôle négatif : TCPS (puits) avec ajout d'une solution de lyse cellulaire. L'ensemble induit une réponse cytotoxique reproductible ;

PLA$_{50}$, connu comme matériau biocompatible et favorisant la prolifération cellulaire. Mise en forme identique au P4 et/ou P3 évalué;.
- Seprafilm (Genzyme S.A.S), barrière commerciale anti-adhérentielle composée d'acide hyaluronique et de carboxyméthylcellulose.

Les cellules utilisées sont une lignée de fibroblastes murine de derme murin CCL 1 (NCTC clone 929), recommandée pour évaluer la cytocompatibilité *in vitro* des dispositifs médicaux selon la norme EN ISO 10993-5 ou une culture de cellules endométriales humaines.

L'ensemble des tests sont réalisés en triplicata pour s'assurer de la reproductibilité des résultats.

### A. *Test d'adhésion cellulaire*

Le test PrestoBlue® permet d'évaluer la viabilité cellulaire par mesure de l'activité métabolique des cellules. Le test (test t Student) est réalisé après mise en contact direct des cellules sur le matériau afin de quantifier les cellules adhérentes.

On mesure par spectrophotométrie à fluorescence des valeurs de fluorescence qui correspondent à la quantité de cellules adhérentes ($\lambda$ Excitation = 531 nm ; $\lambda$ Emission = 615 nm).

Le film P4 utilisé est au préalable immergé dans du PBS à pH 7,4 pendant 24H pour gonfler.

Comme cela est visible sur la figure la, les valeurs de fluorescence reflètent la quantité de cellules ayant adhéré sur le film. On remarque une diminution significative de l'adhésion cellulaire sur le film P4 en comparaison avec le film $PLA_{50}$ et le témoin positif TCPS.

Ainsi, en considérant que 100% des cellules adhèrent sur le témoin positif (TCPS), 69% des cellules adhèrent sur le film de $PLA_{50}$ et seulement 51% des cellules adhèrent sur le film P4 après 45 minutes d'incubation. Les pourcentages sont similaires après 3 heures d'incubation.

La figure 1b confirme la diminution de l'adhésion cellulaire sur les films P4 et les films P3. On observe une diminution de l'adhésion des cellules endométriales humaines sur les films P4 et P3 en comparaison au contrôle positif TCPS après 45 minutes, 1H30 heures et 3 heures d'incubation. On observe également une adhésion cellulaire équivalente sur les films P4, les films P3 et la membrane anti-adhérences Seprafilm® après 3H d'incubation.

### B. *Test de prolifération cellulaire*

Les films P4 et P3 utilisés sont au préalable immergés dans du PBS à pH 7,4 pendant 24H pour gonfler.

L'étude nous montre (figure 2), aux différents jours de prolifération, une diminution notable de la prolifération cellulaire sur le film P4 en comparaison avec le film $PLA_{50}$ et le TCPS. En effet, le film P4 présente des valeurs de fluorescence significativement différentes du $PLA_{50}$ et du TCPS pour des temps de prolifération de 1 et 2 jours.

La prolifération cellulaire diminue également sur le film P3, avec des valeurs de fluorescence significativement plus faibles que pour le TCPS aux différents temps.

L'ensemble des résultats d'adhésion (figures la-lb) et de prolifération (figure 2) montre que le film P4 possède des propriétés qui tendent à diminuer l'adhésion et la prolifération cellulaire. Les propriétés anti-adhérentielles et antiprolifératives du matériau sont dues à la présence de polyéthylène glycol dans la composition.

### C. *Test de cytotoxicité*

Selon la norme EN ISO 10993-5 relative à l'évaluation biologique des dispositifs médicaux, la cytotoxicité peut être évaluée dès 24H d'incubation par une évaluation quantitative :

- mesure du paramètre mort cellulaire.

Le lactate déshydrogénase (LDH) est une enzyme présente dans les cellules eucaryotes qui, lorsqu'il y a mort cellulaire, est libérée dans le milieu. Le kit (LDH Cytotoxicity Assay Kit) est une méthode colorimétrique afin de doser la LDH, marqueur de la cytotoxicité cellulaire. L'activité de la LDH est mesurée par spectrophotométrie à 490 nm après 24H d'incubation des cellules endométriales humaines en présence du matériau.

Les films de P4, P3 et PLA50 utilisés sont au préalable immergés dans du PBS à pH 7,4 pendant 24H pour gonfler.

Comme le montre la figure 3, les films P4, P3 et $PLA_{50}$ ont des valeurs d'absorbance comprises entre le contrôle positif et le contrôle négatif.

De plus, les films P4 et P3 ont une valeur d'absorbance significativement différente du contrôle positif et les copolymères P4, P3, le $PLA_{50}$ et le contrôle négatif sont issus d'une même population (p-value > 0,05 P4-P3 versus PLAso/Contrôle négatif). L'ensemble de ses informations renseigne sur la non-toxicité des films P4 et P3.

4- Evaluation des propriétés de déploiement du matériau anti-adhérentiel

Pour l'ensemble des études de déploiement, les matériaux utilisés sont obtenus à partir d'une composition de copolymères P4 ou à partir d'une composition de copolymères P3, mis en forme par pressage à chaud. Les films obtenus à partir des copolymères ont une épaisseur de 400$\mu$m (mesure obtenue au microscope optique).

Un matériau obtenu à partir d'une composition de copolymères P4 mis en forme par évaporation de solvant a également été réalisé. Le film obtenu a une épaisseur de film de 300 $\mu$m (mesure obtenue au microscope optique)..

Tous les tests sont réalisés en triplicata pour s'assurer de la reproductibilité des résultats.

## A. *Etude de gonflement*

Les films anti-adhérentiels et les films de PLA50 utilisés dans l'étude sont découpés en bandelettes (masse au temps to connue) puis mis à incuber à 37°C en milieu salin (PBS 1X), sous agitation.

La masse des bandelettes est pesée à différents temps et le pourcentage de prise d'eau est calculé comme suit :

$$\text{Prise d'eau } (\%) = \frac{(\text{Masse }(t) - \text{Masse }(t_0))}{(\text{Masse }(t_0))} \times 100$$

Les bandelettes de film P4 gonflent dans le PBS, avec une prise d'eau de l'ordre de 300% dès 5 minutes d'incubation (figure 4a). La teneur en eau au sein du matériau augmente au cours du temps, jusqu'à atteindre un pourcentage de l'ordre de 1000%. Le pourcentage de prise d'eau se stabilise dès le huitième jour d'incubation jusqu'au quatorzième jour d'incubation. Le film P4 se délite ensuite en fragments après quinze jours d'incubation.

Les bandelettes de film P3 gonflent également dans le PBS, avec une prise d'eau de l'ordre de 600% dès le douzième jour d'incubation, et ceux jusqu'au vingt-deuxième jour. Le film de copolymère P3 se délite ensuite après vingt-cinq jours d'immersion dans le PBS.

Le pourcentage de prise d'eau du film P4 obtenu par évaporation de solvant augmente très rapidement à environ 900%, puis se stabilise ensuite dès 24H d'incubation (non représenté).

La capacité des copolymères P4 et P3 à gonfler en milieu aqueux est due au bloc hydrophile de PEG de haut poids moléculaire. L'augmentation du ratio EO/LA dans la composition des copolymères entraîne une augmentation de la prise d'eau.

## B. *Etude d'augmentation de surface*

L'étude suivante permet de déterminer l'évolution de la surface de ces mêmes matériaux.

Les films anti-adhérentiels utilisés dans l'étude sont découpés en bandelettes (surface au temps To connue) puis mis à incuber à 37°C en milieu salin (PBS 1X), sous agitation. Les dimensions des bandelettes sont mesurées à différents temps et on détermine l'augmentation de surface comme suit :

$$\text{Augmentation de surface } (\%) = \frac{(\text{Surface }(t) - \text{Surface }(t_0))}{\text{Surface }(t_0)} \times 100$$

Quelle que soit la mise en forme des bandelettes à base de copolymère P4, celles-ci gonflent dans le PBS et doublent de surface dès 5 minutes d'incubation. Les bandelettes à base de copolymères P3 gonflent dans le PBS et doublent de surface dès 30 minutes d'incubation (figure 4b).

Une mise en forme de P4 par évaporation de solvant permet une augmentation de surface de l'ordre de 300% dès 5H d'incubation, et la surface de la membrane se stabilise ensuite (non représenté).

L'augmentation de surface diffère légèrement avec une mise en forme de P4 par pressage à chaud. En effet, la surface des bandelettes augmente de 250% en 3H d'incubation pour ensuite se stabiliser. Cette différence peut s'expliquer par l'arrangement des chaînes de polymères lors de l'étape de mise en forme.

De la même manière, pour le copolymère P3 l'augmentation de surface est de l'ordre de 150% après 5H d'incubation et la surface se stabilise ensuite.Lorsqu'on mesure à l'aide d'une règle l'augmentation de surface d'une bandelette à base de copolymère P4 mise en forme par évaporation, dont les dimensions « sèches » (à t = 0) sont de 0,5 cm de hauteur et 1 cm de long, on observe une augmentation rapide dans les deux dimensions après seulement 5 minutes d'incubation (t = 5 minutes) : hauteur de 0,8 cm et longueur de 1,6 cm. A t = 30 minutes, la hauteur est de 0,9 cm et la longueur de 1,8 cm, pour atteindre à t = 24h 1 cm de hauteur pour 2 cm de long, et se stabiliser.

## C. *Etude d'augmentation de volume*

L'étude suivante permet de déterminer l'évolution du volume des films P4 et P3 obtenus par pressage à chaud.

Les films anti-adhérentiels utilisés dans l'étude sont découpés en bandelettes (surface au temps To connue) puis mis à incuber à 37°C en milieu salin (PBS 1X), sous agitation. Les dimensions des bandelettes sont mesurées à différents temps et on détermine l'augmentation du volume comme suit :

$$\text{Volume} = \text{Surface} \times \text{Epaisseur}$$

$$\text{Augmentation de volume (\%)} = \frac{(\text{Volume (t)} - \text{Volume (t}_0))}{\text{Volume (t}_0)} \times 100$$

Le volume des films de copolymères P3 et de copolymères P4 augmente au cours du temps d'immersion en milieu aqueux (figure 4c). Le volume des films de copolymères P3 triple dès 2H d'incubation jusqu'à avoir un volume multiplié par un facteur 10 après 16 jours d'incubation. L'augmentation du volume des films de copolymères P4 est plus rapide en comparaison au film de copolymères P3, avec un volume multiplié par un facteur 10 dès 5 jours d'incubation.

## D. *Etude de déploiement*

Le gonflement du matériau mis en forme à partir d'une composition de copolymères P4 induit un déploiement de la membrane en milieu aqueux.

Le déploiement d'un film P4 peut être illustré par l'expérimentation ci-dessous (figures 5a-5h):
Une bandelette rectangulaire d'un film P4, mis en forme par pressage à chaud, de longueur 4 cm et de largeur 2,4 cm, est enroulée en escargot puis immergée dans de l'eau. On visualise, par microscopie optique, le déploiement de la bandelette ainsi que l'évolution de son épaisseur.

Avant son immersion dans l'eau, l'épaisseur de la bandelette ainsi que son diamètre une fois enroulée en escargot sont mesurés (dimensions sèches): épaisseur initiale (to) = 463 $\mu$m ; diamètre initial (to) = 4724 $\mu$m.

Le suivi du déploiement et de l'évolution de l'épaisseur est réalisé jusqu'à t= 24H, le gonflement du copolymère se stabilisant dès 24H d'incubation.

Les figures 5a à 5h permettent de visualiser la progression du déploiement de la bandelette une fois immergée dans l'eau. La bandelette se déroule au cours du temps, avec dès les 2 premières minutes dans l'eau une augmentation du volume occupé (de to = 4 784 $\mu$m à $t_{2min}$ = 5 625 $\mu$m). La bandelette se déploie pour complètement occuper l'ensemble de l'espace disponible dès 15 minutes d'immersion ($t_{15\ min}$ = 9 655 $\mu$m). Le phénomène de déploiement se concrétise avec une longueur totale qui atteint 10 634 $\mu$m à 24h, soit plus du double de la longueur initiale.

Le déploiement de la bandelette est la conséquence du gonflement du copolymère dans l'eau. Le gonflement se traduit par une augmentation de l'épaisseur au cours du temps, avec une épaisseur maximale à 24H de 1 312$\mu$m, soit une augmentation d'environ 185% par rapport à l'épaisseur initiale.

Le phénomène de déploiement de la bandelette est principalement lié à la capacité du copolymère à absorber une importante quantité d'eau. Le copolymère gonfle et possède ainsi la force de se déployer et d'occuper l'espace disponible.

## 5- Evaluation du déploiement et de l'occupation de l'espace disponible

Un film a été réalisé par évaporation de solvant d'une composition de copolymères P4 et découpé en trapèze, pour être ensuite introduit dans un applicateur à stérilet.

Le film en trapèze est expulsé de l'applicateur dans un modèle mimant une cavité utérine remplie d'eau, au moyen d'un système de poussoir afin de s'y déployer (figure 6). Le modèle de cavité utérine possède les dimensions théoriques d'une cavité utérine humaine.

Une fois le film expulsé dans la cavité, celle-ci prend une dizaine de minutes pour se déployer et recouvrir la totalité de la cavité (figure 7a à 7e).

6- Evaluation de la dégradation du matériau anti-adhérentiel

Afin de déterminer la dégradation *in vitro* des films anti-adhérentiels mis en forme de film par pressage à chaud à partir de la composition P4 ou P3, on introduit un rectangle (1,5 x 1 cm) découpé dans lesdits films dans du PBS, à 37°C, sous agitation mécanique.

Les propriétés mécaniques des films P4 et P3 sont évaluées à différents temps d'incubation par des mesures rhéologiques.

La figure 8a montre le module élastique G' et le module visqueux G" des films P3 et P4 après 5 minutes d'immersion, lorsqu'on leur applique une gamme de déformation à fréquence fixe (1Hz). Pour les deux copolymères, le module élastique est supérieur au module visqueux, caractéristique d'un matériau solide élastique.

La figure 8b montre la variation du module élastique (G') et du module visqueux (G") en fonction de la fréquence (à 0,05% de déformation) pour les films P4 et P3 après 2 jours et 21 jours d'immersion. Pour les deux copolymères, les valeurs des modules diminuent au cours de la période d'incubation, caractéristique d'une dégradation des copolymères. Par exemple, pour une fréquence de 2Hz, G'(P3) passe d'une valeur de 2005 Pa à 739 Pa après 2 jours et 21 jours respectivement. La perte des propriétés mécaniques est directement reliée à la dégradation du film.

La figure 8c montre le module élastique (G') à différents temps d'incubation pour les films P4 et P3. Les valeurs de G' permettent de mettre en évidence une cinétique exponentielle d'ordre 1, avec une cinétique de dégradation plus rapide pour le film P4 que pour le film P3.

La figure 8d montre la contrainte du film P4 après 30 jours d'incubation et la contrainte du film P3 après 40 jours d'incubation en fonction du taux de cisaillement. La figure montre que les matériaux ont un degré de viscosité élevé, avec une contrainte critique ($\square_c$) d'environ 0,25 pour le P3 et 0,35 pour le P4.

La dégradation in vitro est également illustrée par la figure 9 qui montre le pourcentage de perte de masse des films de copolymères P3, des films P4 et des films composés de PLA50 au cours du temps. Les films de copolymères perdent de leur masse jusqu'à une solubilisation complète des films après 90 jours d'incubation.

Les figures 10a-10e illustrent le film P4 à différents temps de dégradation. Celui-ci maintient son intégrité physique pendant les premiers jours de son immersion, à savoir un film malléable et élastique. La perte de ses propriétés mécaniques apparait nettement après 12 jours d'immersion, avec un film qui se délite lorsqu'il est manipulé. Cette observation permet de mettre en évidence la dégradation du film. A 40 jours, le film est complètement fragmenté.

Les figures 10a' à 10c' illustrent quant à elles les propriétés filmogènes du film P3 (figure 10a') à différents temps de dégradation. Le film P3 maintient ses propriétés mécaniques après 30 jours d'immersion (figure 10b'), puis celui-ci se délite en morceaux de gel après 60 jours d'immersion (figure 10c').

La dégradation du copolymère est également mise en évidence par l'analyse RMN $^1$H qui indique la formation d'acide lactique après 1 mois et deux semaines d'incubation dans le PBS. La présence d'acide lactique est un marqueur de l'hydrolyse des chaînes de poly(acide lactique) au sein du matériau.

La dégradation in vitro du copolymère P4 est mise en évidence par la perte de masse Mw (Figure 11) et l'augmentation de sa dispersité, obtenue par Chromatographie d'Exclusion Stérique. La Mw chute au cours du temps, avec une perte de près de 50% dès 20 jours d'incubation. Cette diminution s'explique par l'hydrolyse des chaînes de PLA. Ses chaînes de masses molaires de plus en plus faibles sont hydrolysées pour ensuite atteindre le stade d'oligomères puis *in fine* celui de monomères d'acide lactique.

Afin de déterminer la dégradation *in vivo* des films de copolymères P4 et de copolymères P3, des rectangles (4 x 3,5 cm) découpés dans lesdits films sont introduits entre le caecum et le péritoine d'une ratte. Un defect est effectué sur le péritoine et le caecum et est abrasé à la compresse pour créer des adhérences entre les organes, et le film de copolymère est immédiatement appliqué entre le caecum abrasé et le defect péritonéal avant la fermeture abdominale. Les rattes sont euthanasiées à différents temps après le geste opératoire.

La figure 12a montre le film de copolymère P4 avant son introduction dans l'animal (t = 0), et la figure 12b montre les résidus de copolymères P4 récupérés après 2 jours d'implantation. On observe une dégradation du film de copolymère P4 dès 2 jours post-opératoires en particules de gel, avec une grande partie du film initial solubilisé et éliminé de l'organisme.

Les figures 12b' à 12e' montrent le film de copolymère P3 (film intègre avant implantation : figure 12a') après différents temps de résidence dans la cavité abdominale de l'animal. La figure 12b' et la figure 12c' montrent des fragments de film P3 récupérés après respectivement 12H et 2 jours de résidence dans l'organisme de l'animal. La figure 12d' montre le film P3 en petits morceaux de film après 5 jours de résidence et la figure 12e' montre le film P3 en particules de gel après 12 jours, avec la majeure partie du film initial solubilisé et éliminé de la cavité abdominale.

7- Evaluation de l'efficacité anti-adhérentielle des copolymères P4 et P3 sur un modèle d'adhérences péritonéales de rats

*Matériels et méthodes*

34 femelles Wistar sont anesthésiées après injection musculaire de Ketamine (50mg/kg) et de xylazine (5mg/kg). L'abdomen est désinfecté à la Bétadine et une anesthésie locale sous cutanée est réalisée avant incision de l'abdomen (Lidocaine 0,1%).

On réalise une incision médiane verticale de l'abdomen sur 4 cm de long puis on procède à l'ouverture de la cavité abdominale. La séreuse caecale est abrasée à la compresse et réalisation d'une pastille de péritoine en regard (1cm$^2$). Les films de copolymères et le Seprafilm® sont directement placés dans le flanc entre le cæcum et le péritoine et 1 ml de sérum physiologique est instillé dans la cavité abdominale avant fermeture. Pour le Hyalobarrier®, le gel (environ 1ml) est instillé sur le péritoine et sur le caecum traumatisé. Pour le groupe contrôle, l'abdomen est directement refermé (Vicryl 3-0 Ethicon resorbable) après abrasion du cæcum et réalisation de la pastille de péritoine. Une injection de Buprénorphine (0,02mg/kg) est réalisée de manière systématique dans les 48 premières heures suivant la procédure chirurgicale.

L'étude d'efficacité comporte 5 groupes:

Groupe A (n=6) : Avec film de copolymères P4

Groupe B (n=6) : Avec film de copolymères P3

Groupe C (n=6): Avec Seprafilm®; Référence 4301-03

Groupe D (n=6): Avec Hyalobarrier®

Groupe E (n=6): Témoin

Le score adhérentiel est évalué selon l'extension, la sévérité et le degré des adhérences de la façon suivante :

1) évaluation de l'extension des adhérences:

0 = pas d'adhésion

1 = adhérence concernant moins de 25% de la surface traumatisée

2 = adhérence concernant entre 25 et 50% de la surface traumatisée

3 = adhérence concernant entre 50 et 75% de la surface traumatisée

4 = adhérence concernant entre 75 et 100% de la surface traumatisée

2) évaluation de la sévérité des adhérences:

0 = pas d'adhésion

1 = adhérence fine avasculaire

2 = adhérence vascularisée opaque

3 = adhérence totale du cæcum à la paroi péritonéale

3) évaluation du degré d'adhérences:

0 = pas d'adhérence

1 = adhérence séparée du tissu par une traction douce

2 = adhérence séparée du tissu par une traction modérée

3 = adhérence nécessitant une traction forte pour être libérée ou impossibilité de libérer sans lésion d'organe
SCORE: / 10

3 rattes de chaque groupe sont sacrifiées à 5 jours suivant la chirurgie et 3 rattes sont ensuite sacrifiées à 12 jours pour l'évaluation du score adhérentiel entre le caecum et le péritoine.

4 rattes supplémentaires sont utilisées pour évaluer le score adhérentiel et le comportement des copolymères à des temps précoces : évaluation du P3 à 12H et 2 jours post-opératoire, et du P4 à 2 jours post-opératoire. 1 ratte est utilisée pour évaluer la présence/l'absence d'adhérence à J2 post-opératoire.

La mise à mort des animaux est réalisée par anesthésie de kétamine puis injection létale de Pentobarbital.

Mise en forme des films de copolymères :

Les films de copolymères sont mis en forme par pressage à chaud, et colorés via un colorant violet pour permettre une meilleure visualisation du film dans la cavité abdominale lors du sacrifice de l'animal. Les films de copolymères ont une épaisseur de 400 µm et sont découpés en rectangle de 4cm*3,5cm (l*L). Les films sont stérilisés par irradiation gamma puis conservés au congélateur jusqu'au jour de la chirurgie. Les films ne nécessitent pas d'hydratation au préalable, ni de technique de fixation dans la cavité.

2 jours après l'intervention, la ratte contrôle ne présente aucune adhérence. Il en est de même pour les rattes ayant reçu un film de P4 et de P3. L'étude informe sur le temps de formation des adhérences caecum/péritoine avec l'absence d'adhérence à J2 post-opératoire. Le film de P3 est présent à J2 (figure 11c') au niveau de la zone traumatisée, alors que le film P4 est dégradé avec seulement des petites particules de gel dans la cavité (figure 11b). En l'absence d'adhérence chez la ratte contrôle, on ne peut conclure sur l'efficacité anti-adhérentiel des copolymères à J2.

5 jours après l'intervention chirurgicale, 3 rattes de chaque groupe sont sacrifiées. Dans le groupe contrôle, les rattes possèdent des adhésions sévères (*Tableau 1*) qui concernent entre 75% et 100% de la surface péritonéale (extension = 4), avec l'adhérence totale du cæcum à la paroi péritonéale (sévérité = 3) et l'impossibilité de libérer le cæcum sans lésion de l'organe (degré = 3). Après application de Seprafilm®, on observe des adhérences à J5 avec un score moyen de 7,33. Dans le groupe ayant reçu le hyalobarrier®, le score est nul. Aucune adhérence n'est observée, preuve de son efficacité à J5 post-opératoire.

Dans le groupe avec le film de copolymère P4, 2 rattes présentent des adhésions, la 3ème présente une cavité indemne. Avec un score moyen de 4,66, le score adhérentiel reste nettement inférieur au score du groupe contrôle.

Dans le groupe avec le film de copolymère P3, aucune adhésion n'est observée (score nul) et le film est en petits morceaux (figure 11d') dans la cavité.

Tableau 1 : Evaluation de l'efficacité anti-adhérentielle après 5 jours post-opératoire.

| | Rat | Extension | Sévèrité | Degré | Score adhérentiel | Moyenne Score adhérentiel |
|---|---|---|---|---|---|---|
| **Groupe A: Copolymère P4** | 1 | 4 | 3 | 1 | 8 | |
| | 2 | 1 | 2 | 3 | 6 | 4,66 |
| | 3 | 0 | 0 | 0 | 0 | |
| **Groupe B: Copolymère P3** | 7 | 0 | 0 | 0 | 0 | |
| | 8 | 0 | 0 | 0 | 0 | 0 |
| | 9 | 0 | 0 | 0 | 0 | |
| **Groupe C : Seprafilm ®** | 13 | 4 | 3 | 3 | 10 | |
| | 14 | 1 | 2 | 2 | 5 | 7,33 |
| | 15 | 3 | 2 | 2 | 7 | |
| **Groupe D : Hyalobarrier** | 19 | 0 | 0 | 0 | 0 | |
| | 20 ® | 0 | 0 | 0 | 0 | 0 |
| | 21 | 0 | 0 | 0 | 0 | |
| **Groupe E : Témoin** | 25 | 4 | 3 | 3 | 10 | |
| | 26 | 4 | 3 | 3 | 10 | 10 |
| | 27 | 4 | 3 | 3 | 10 | |

12 jours après l'intervention chirurgicale, les 3 rattes restantes de chaque groupe sont à leurs tours sacrifiées et le score adhérentiel est évalué (*Tableau 2*). Le score adhérentiel à J12 pour le groupe contrôle est élevé (score 8,6), caractéristiques d'adhérences vascularisées recouvrant chez l'ensemble des rattes au moins 50% de la surface traumatisée.

Après application de Seprafilm®, nous observons des adhérences à J12 avec un score moyen de 4,3. La mise en place du film n'est pas simple, puisque celui-ci est extrêmement friable. Au vu des résultats, l'efficacité anti-adhérentielle n'est pas démontrée.

Dans le groupe ayant reçu le Hyalobarrier®, 2 rattes présentent des adhérences d'intensité comparables au groupe témoin, avec l'accolement du caecum au péritoine avec l'impossibilité de décoller le caecum sans lésion de l'organe. La 3ème ratte du groupe possède une cavité indemne. Ainsi, Hyalobarrier® joue un rôle anti-adhérentiel à court terme (J0 à J5) puis le gel est dégradé notamment par hydrolyse enzymatique, et ne permet pas un rôle barrière sur une période de 12 jours (score =6.6).

Pour le groupe avec le film de copolymère P4, le score adhérentiel est similaire à celui observé à J5. Le score adhérentiel du copolymère P4 (score=5.3) est compris entre le score adhérentiel des produits concurrents Hyalobarrier® et Seprafilm®. Du fait de la dégradation précoce du film de copolymère P4, le film ne permet pas un effet anti-adhérentiel sur le long terme.

Dans le groupe avec le film de copolymère P3, aucune adhésion n'est observée (score nul), et le péritoine est macroscopiquement cicatrisé. Le copolymère P3 est retrouvé sous la forme de particules de gels (figure 11e'). L'efficacité anti-adhérentiel du P3 est sans équivoque.

Tableau 2 : Evaluation de l'efficacité anti-adhérentielle après 12 jours post-opératoire.

| | Rat | Extension | Sévèrité | Degré | Score adhérentiel | Moyenne Score adhérentiel |
|---|---|---|---|---|---|---|
| **Groupe A: Copolymère P4** | 4 | 0 | 0 | 0 | 0 | |
| | 5 | 2 | 3 | 3 | 8 | 5,3 |
| | 6 | 2 | 3 | 3 | 8 | |
| **Groupe B : Copolymère P3** | 10 | 0 | 0 | 0 | 0 | |
| | 11 | 0 | 0 | 0 | 0 | 0 |
| | 12 | 0 | 0 | 0 | 0 | |
| **Groupe C : Seprafilm ®** | 16 | 1 | 1 | 1 | 3 | |
| | 17 | 4 | 3 | 3 | 10 | 4,3 |
| | 18 | 0 | 0 | 0 | 0 | |
| **Groupe D : Hyalobarrier ®** | 22 | 4 | 3 | 3 | 10 | |
| | 23 | 4 | 3 | 3 | 10 | 6,6 |
| | 24 | 0 | 0 | 0 | 0 | |
| **Groupe E : Témoin** | 28 | 4 | 3 | 3 | 10 | |
| | 29 | 3 | 2 | 1 | 6 | 8,6 |
| | 30 | 4 | 3 | 3 | 10 | |

Dans le groupe contrôle, le geste opératoire traumatisant pour la cavité abdominale entraîne des adhésions sévères entre le péritoine et le caecum lésé. L'application du film de copolymère P4 en post-opératoire permet une réduction de la sévérité des adhésions. L'application du film de copolymère P3 permet l'absence totale d'adhérence.

**Revendications**

**1.** Composition à base de copolymères comprenant au moins un copolymère blocs A et B, dans lequel :

- le bloc A est un polyester ;
- le bloc B est un poly-oxyéthylène (PEG) ;
- la masse moléculaire en poids des blocs B, déterminée par chromatographie d'exclusion stérique réalisée dans du diméthylformamide en tant que solvant d'analyse, en utilisant une gamme étalon de Poly(éthylène glycol), est supérieure ou égale à 50 kDa ; et
- le ratio molaire motif éthylène oxyde/motif ester est compris entre 0,5 et 5.

**2.** Composition à base de copolymères selon la revendication 1, dans laquelle le copolymère blocs A et B est choisi parmi les copolymères diblocs AB et triblocs ABA et BAB, et leurs mélanges.

3. Composition à base de copolymères selon la revendication 1 ou 2, dans laquelle la masse moléculaire en poids des blocs B dans le copolymère est comprise entre environ 75 kDa et environ 150 kDa, de manière préférée entre environ 80 et environ 125 kDa, plus préférentiellement entre environ 90 et environ 115 kDa, de manière encore préférée entre environ 100 kDa et environ 110 kDa.

4. Composition à base de copolymères selon l'une quelconque des revendications précédentes, dans laquelle le ratio motif éthylène oxyde/motif ester est de 1 à 3.

5. Composition à base de copolymères selon l'une quelconque des revendications précédentes, dans laquelle les blocs A sont des poly (acides lactiques) choisis notamment parmi le poly (acide L-lactique), le poly (acide D-lactique) et le poly (acide D,L lactique).

6. Composition à base de copolymères selon la revendication 5, dans laquelle le PLLA représente au moins 50% en poids des blocs A, et préférentiellement de 75% à 100% en poids des blocs A.

7. Composition à base de copolymères selon l'une quelconque des revendications précédentes, ladite composition étant destinée à un usage médical, et préférentiellement pour la prévention des synéchies intra-utérines.

8. Matériau anti-adhérentiel obtenu par mise en forme de la composition de copolymères selon l'une des revendications 1 à 7, où par propriétés anti-adhérentielles on entend que le matériau est apte à prévenir ou à tout le moins limiter la prolifération cellulaire et l'adhésion cellulaire à sa surface, comparativement à un matériau ne présentant pas de telles propriétés.

9. Matériau anti-adhérentiel selon la revendication 8, dans lequel ledit matériau est mis en forme par extrusion, évaporation par solvant, pressage à chaud ou imprimante 3D.

10. Matériau anti-adhérentiel selon la revendication 8 ou 9, sous la forme d'un film présentant en milieu aqueux un taux de gonflement de 1 à 20, préférentiellement de 3 à 15 et plus préférentiellement au moins égal à 4, où le taux de gonflement est déterminé comme indiqué dans la description.

11. Matériau anti-adhérentiel selon l'une des revendications 8 à 10, dans lequel les copolymères se dégradent après un temps de séjour en milieu aqueux compris entre 2 et 20 jours, et préférentiellement compris entre 3 et 15 jours.

12. Matériau anti-adhérentiel selon l'une des revendications 8 à 9 , dans lequel ledit matériau comprend en outre un additif ou principe actif additionnel, tel qu'une molécule anti-adhérentielle ou une molécule à visée thérapeutique.

13. Dispositif médical destiné à la prévention des synéchies intra-utérines comprenant le matériau anti-adhérentiel selon l'une des revendications 8 à 12.

14. Dispositif médical selon la revendication 13, dans lequel le matériau anti-adhérentiel est sous la forme d'un film en trapèze destiné à se déployer dans la cavité utérine pour en épouser le contour et s'accoler aux parois.

15. Dispositif médical selon l'une des revendications 13 ou 14, comprenant des moyens d'insertion du matériau anti-adhérentiel dans la cavité utérine.

**Patentansprüche**

1. Zusammensetzung auf Basis von Copolymeren, umfassend wenigstens ein AB-Blockcopolymer, wobei

- Block A ein Polyester ist;
- Block B ein Polyoxyethylen (PEG) ist;
- das Molekulargewicht der B-Blöcke, bestimmt mittels Größenausschluss-Chromatographie, durchgeführt in Dimethylformamid als Lösemittel der Analyse, unter Verwendung einer Reihe an Polyethylenglycol-Standards, größer oder gleich 50 kDa ist; und
- das Molverhältnis Ethylenoxid-Einheit/Ester-Einheit zwischen 0,5 und 5 beträgt.

2. Zusammensetzung auf Basis von Copolymeren gemäß Anspruch 1, wobei das AB-Blockcopolymer aus AB-Diblockcopolymeren und ABA- und BAB-Triblockcopolymeren und ihren Gemischen ausgewählt ist.

3. Zusammensetzung auf Basis von Copolymeren gemäß Anspruch 1 oder 2, wobei das Molekulargewicht der B-Blöcke in dem Copolymer zwischen ungefähr 75 kDa und ungefähr 150 kDa, bevorzugt zwischen ungefähr 80 kDa und ungefähr 125 kDa, bevorzugter zwischen ungefähr 90 kDa und ungefähr 115 kDa, insbesondere bevorzugt zwischen ungefähr 100 kDa und ungefähr 110 kDa beträgt.

4. Zusammensetzung auf Basis von Copolymeren gemäß einem der vorhergehenden Ansprüche, wobei das Verhältnis Ethylenoxid-Einheit/Ester-Einheit 1 bis 3 beträgt.

5. Zusammensetzung auf Basis von Copolymeren gemäß einem der vorhergehenden Ansprüche, wobei die A-Blöcke Polymilchsäuren sind, ausgewählt insbesondere aus Poly-L-milchsäure, Poly-D-milchsäure und Poly-D,L-milchsäure.

6. Zusammensetzung auf Basis von Copolymeren gemäß Anspruch 5, wobei PLLA wenigstens 50 Gew.-% der A-Blöcke und bevorzugt 75 Gew.-% bis 100 Gew.-% der A-Blöcke darstellt.

7. Zusammensetzung auf Basis von Copolymeren gemäß einem der vorhergehenden Ansprüche, wobei die Zusammensetzung für eine medizinische Verwendung und bevorzugt für die Vorbeugung intrauteriner Synechien bestimmt ist.

8. Antiadhäsionsmaterial, erhalten durch Formung der Zusammensetzung von Copolymeren gemäß einem der Ansprüche 1 bis 7, wo unter antiadhäsiven Eigenschaften zu verstehen ist, dass das Material geeignet ist, die Zellproliferation und die Zelladhäsion auf seiner Oberfläche zu verhindern oder wenigstens zu begrenzen verglichen mit einem Material, das derartige Eigenschaften nicht aufweist.

9. Antiadhäsionsmaterial gemäß Anspruch 8, wobei das Material mittels Extrusion, Lösemittel-Verdampfung, Heißpressen oder 3D-Druck geformt wird.

10. Antiadhäsionsmaterial gemäß Anspruch 8 oder 9 in Form eines Films, der in wässrigem Milieu ein Quellverhältnis von 1 bis 20, bevorzugt von 3 bis 15, bevorzugter wenigstens gleich 4 aufweist, wobei das Quellverhältnis, wie in der Beschreibung angegeben, bestimmt wird.

11. Antiadhäsionsmaterial gemäß einem der Ansprüche 8 bis 10, wobei die Copolymere sich nach einer Verweildauer in wässrigem Milieu zwischen 2 und 20 Tagen und bevorzugt zwischen 3 und 15 Tagen abbauen.

12. Antiadhäsionsmaterial gemäß einem der Ansprüche 8 bis 9, wobei das Material außerdem einen weiteren Zusatz- oder Wirkstoff wie ein antiadhäsives Molekül oder ein therapeutisches Molekül umfasst.

13. Medizinische Vorrichtung zur Vorbeugung intrauteriner Synechien, umfassend das Antiadhäsionsmaterial gemäß einem der Ansprüche 8 bis 12.

14. Medizinische Vorrichtung gemäß Anspruch 13, wobei das Antiadhäsionsmaterial in Form eines trapezförmigen Films vorliegt, der sich in der Uterushöhle entfalten soll, um sich deren Konturen anzupassen und an den Wänden anzuliegen.

15. Medizinische Vorrichtung gemäß einem der Ansprüche 13 oder 14, umfassend Mittel zum Einführen des Antiadhäsionsmaterials in die Uterushöhle.

**Claims**

1. Composition based on copolymers comprising at least one A B blocks copolymer, wherein:

   - the A block is a polyester;
   - the B block is a poly-oxyethylene (PEG);
   - the molecular mass in weight of the B blocks, determined by size-exclusion chromatography carried out in dimethylformamide as analysis solvent, using a standard range of polyethylene glycol, is greater than or equal to 50 kDa; and
- the molar ratio of ethylene oxide units to ester units is between 0.5 and 5.

2. Composition based on copolymers according to claim 1, wherein the A B blocks copolymer is chosen among the AB diblocks and ABA and BAB triblocks copolymers, and the mixtures thereof.

3. Composition based on copolymers according to claim 1 or 2, wherein the molecular mass in weight of the B blocks in the copolymer is between about 75 kDa and about 150 kDa, preferably between about 80 and about 125 kDa, more preferably between about 90 and about 115 kDa, still more preferably between about 100 kDa and about 110 kDa.

4. Composition based on copolymers according to any one of the preceding claims, wherein the ratio of ethylene oxide units to ester units is between 1 and 3.

5. Composition based on copolymers according to any one of the preceding claims, wherein the A blocks are polylactic acids chosen, in particular, from poly-L-lactic acid, poly-D-lactic acid and poly-DL-lactic acid.

6. Composition based on copolymers according to claim 5, in which PLLA represents at least 50% by weight of the A blocks, and preferably from 75% to 100% by weight of the A blocks.

7. Composition based on copolymers according to any one of the preceding claims, said composition being intended for a medical use, and preferably for the prevention of intrauterine synechiae.

8. Anti-adhesion material obtained by shaping the copolymer composition according to one of claims 1 to 7, wherein anti-adhesion properties means that the material is capable of preventing or at the very least limiting cellular proliferation and cellular adhesion at its surface, compared with a material not having such properties.

9. Anti-adhesion material according to claim 8, wherein said material is shaped by extrusion, solvent evaporation, hot pressing or 3D printing.

10. Anti-adhesion material according to claim 8 or 9, in the form of a film having, in aqueous medium, a swelling ratio of 1 to 20, preferably 3 to 15 and more preferably at least equal to 4, wherein the swelling ratio is determined as indicated in the description.

11. Anti-adhesion material according to one of claims 8 to 10, wherein the copolymers degrade after a residence time in aqueous medium of between 2 and 20 days, and preferably between 3 and 15 days.

12. Anti-adhesion material according to one of claims 8 to 9, wherein said material further comprises an additive or additional active ingredient, such as an anti-adhesion molecule or a molecule for therapeutic purposes.

13. Medical device intended for the prevention of intrauterine synechiae comprising the anti-adhesion material according to one of claims 8 to 12.

14. Medical device according to claim 13, wherein the anti-adhesion material is in the form of a trapezoidal film intended to be deployed in the uterine cavity in order to mold to the contours and to be juxtaposed with the walls thereof.

15. Medical device according to one of claims 13 or 14, comprising means for inserting the anti-adhesion material into the uterine cavity.

a

b

FIGURE 1

FIGURE 2

FIGURE 3

FIGURE 4

FIGURE 5

FIGURE 6

FIGURE 7

FIGURE 8

FIGURE 8 (suite)

FIGURE 9

FIGURE 10

FIGURE 11

FIGURE 12

FIGURE 13

FIGURE 14

FIGURE 15

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 7202281 B